(19) Europäisches Patentamt
European Patent Office
Office européen des brevets

(11) **EP 2 871 463 B1**

(12) **EUROPÄISCHE PATENTSCHRIFT**

(45) Veröffentlichungstag und Bekanntmachung des Hinweises auf die Patenterteilung:
**21.02.2018 Patentblatt 2018/08**

(51) Int Cl.:
*G01N 21/47* (2006.01) *G01N 33/543* (2006.01)

(21) Anmeldenummer: **14192873.9**

(22) Anmeldetag: **12.11.2014**

(54) **Vorrichtung und Verfahren zur Untersuchung eines oder mehrerer Phasenobjekte**

Device and method for analysing one or more phase objects

Procédé et dispositif destinés à l'examen d'un ou plusieurs objets de phase

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.11.2013 DE 102013112415**

(43) Veröffentlichungstag der Anmeldung:
**13.05.2015 Patentblatt 2015/20**

(73) Patentinhaber: **Ludwig-Maximilians-Universität München**
**80539 München (DE)**

(72) Erfinder:
• **Paulitschke, Philipp**
**81371 München (DE)**
• **Rädler, Joachim**
**81245 München (DE)**

(74) Vertreter: **Lucke, Andreas**
**Boehmert & Boehmert**
**Anwaltspartnerschaft mbB**
**Pettenkoferstrasse 22**
**80336 München (DE)**

(56) Entgegenhaltungen:
**DE-A1-102010 007 365     DE-A1-102011 050 493**
**US-A1- 2002 037 593     US-A1- 2012 231 489**

**Beschreibung**

GEBIET DER ERFINDUNG

Die vorliegende Erfindung liegt auf dem Gebiet der Mikro- oder Nanosensorik. Genauer betrifft die Erfindung eine Vorrichtung und ein Verfahren zur Untersuchung eines oder mehrerer Phasenobjekte, wie beispielsweise biologischer Zellen oder Kleinsttieren. Anwendungen der Vorrichtung und des Verfahrens liegen beispielsweise in der Krebsforschung, Pharmaforschung, Immunologie, Entzündungsmedizin, Embryologie und Biotechnologie.

HINTERGRUND DER ERFINDUNG UND VERWANDTER STAND DER TECHNIK

Aus dem Stand der Technik sind Sensoren bekannt, um die von einer biologischen Zelle ausgeübte Kraft zu messen. Beispielsweise offenbart die DE 10 2011 050 493 A1 einen Zellkraftsensor, der eine Mehrzahl elastischer Elemente umfasst, die jeweils an ihrem unteren Ende an einem Substrat befestigt sind und ein oberes freies Ende aufweisen. Die Auslenkung der elastischen Elemente wird anhand eines Beugungsbildes ermittelt. Ein derartiger Sensor erfordert jedoch einen Aufbau mit elastischen Elementen, der im Einzelfall nur mit erhöhtem Aufwand herzustellen ist. Darüber hinaus ist in der US 2005/0068543 A1 ein System zum Detektieren der Anwesenheit eines Analyten beschrieben. Dabei sind zwei übereinander versetzt angeordnete Gitter vorgesehen. Auf einem der Gitter ist ein Erkennungsmaterial für den Analyten angeordnet. Das Erkennungsmaterial bindet den Analyten an sich. Wenn der Analyt an das Gitter gebunden ist, verändert sich die optische Tiefe der Modulation durch das Gitter. Allerdings ist dieser Aufbau nicht dazu geeignet, ein transientes Verhalten, wie beispielsweise eine Bewegung von biologischen Zellen, mit kleiner Zeitauflösung zu untersuchen.
Gerade die Bestimmung der Bewegung und des Wachstums bzw. Populationswachstums von biologischen Zellen ist jedoch für eine verbesserte Diagnostik, beispielsweise von Krebserkrankungen, wünschenswert. Aktuell bilden Krebserkrankungen die zweithäufigste Todesursache. Bis 2030 soll sich laut der amerikanischen Krebsgesellschaft die Zahl der weltweiten Krebserkrankungen noch fast verdoppeln. Die Beweglichkeit von Zellen, in Fachkreisen Zellmotilität genannt, ist u.a. ein wichtiger Indikator in der Wundheilung, Immunantwort, Angiogenese und bei vielen krankheitsbezogenen Veränderungen, insbesondere bei der Metastasierung im Falle von Krebs. Eine automatisierte Messung der Zellmotilität ist daher von großer Bedeutung für die Entwicklung von medizinischen Wirkstoffen.

Bisher wurden zur Beurteilung der Zellmotilität Verfahren eingesetzt, die auf einer Bildanalyse von Bewegungssequenzen einzelner Zellen und einem Bestimmen der mittleren quadratischen Verschiebung der Zelle pro Zeit beruhen. Häufig werden auch Zellen in 3D-Hydrogelen untersucht, die eine künstliche Gewebematrix darstellen. Diese Verfahren sind jedoch kosten- und zeitintensiv, insbesondere wenn eine größere Anzahl von Zellen für eine statistische Auswertung beobachtet werden soll.

In der DE 10 2010 007365 A1 wird ein Lichtvorhang aus polychromatischem Licht im Infrarotbereich verwendet, um ein Objekt zu detektieren. Zusätzlich zu der Detektion, ob sich überhaupt ein Objekt im Lichtvorhang befindet, wird das Spektrum des von dem Objekt reflektierten Lichts bzw. des transmittierten Lichts mithilfe eines Spektralgitters 17 ermittelt, um Aufschluss über die chemische Zusammensetzung des Objektes zu erhalten.

In der US 2012/231489 A1 wird offenbart, ein aus Phosphorlipiden gebildetes Gitter durch Wechselwirkung mit Proben oder Flüssigkeiten strukturell zu ändern, und diese Änderung durch spektral aufgespaltenes Weißlicht zu detektieren. Konkret werden drei mögliche dynamische strukturelle Änderungen des Gitters betrachtet, die als "spreading", "intercalation" und "dewetting" bezeichnet werden. In diesem Zusammenhang wird aber nicht die Modulation des Lichtfeldes durch Phasenobjekte untersucht, sondern das Gitter wird durch Wechselwirkung mit Proben oder Flüssigkeiten strukturell geändert, und diese strukturelle Änderung wird untersucht, indem weißes, inkohärentes Licht am Gitter aufgespalten und das Spektrum analysiert wird. In einer weiteren Ausdrucksform kommt eine irisierende Oberfläche zum Einsatz, d.h. eine Fläche, bei der sich die Farbe des gebeugten Lichts abhängig von dem Einstrahlungswinkel ändert. Basierend auf der Farbe des gebeugten Lichts für unterschiedliche Einstrahlwinkel wird Aufschluss über die Anhaftung eines Objektes auf der irisierenden Oberfläche gewonnen. Auch bei dieser Analyse wird inkohärentes Weißlicht eingestrahlt.

Die US 2002/037593 A1 offenbart einen optischen biologischen Detektor mit einem Antikörper-Gitter auf einer Siliziumoberfläche. Das Antikörpergitter selbst bildet kein optisches Beugungsgitter. Erst wenn bestimmte Target-Zellen an den Antikörpern andocken, bildet sich ein optisches Beugungsgitter aus.

KURZER ABRISS DER ERFINDUNG

Der Erfindung liegt daher die Aufgabe zugrunde, ein Verfahren und eine Vorrichtung zur Untersuchung von Phasenobjekten, insbesondere einer Zellmotilität, mit hoher Zeitauflösung, hoher Flexibilität und mit geringem Aufwand bereitzustellen, wobei gleichzeitig eine große Anzahl von Phasenobjekten untersucht werden kann.

Diese Aufgabe wird durch ein Verfahren gemäß Anspruch 1 und eine Vorrichtung gemäß Anspruch 24 gelöst. Vorteilhafte Weiterbildungen sind in den abhängigen Ansprüchen angegeben.

In einem ersten Aspekt wird ein Verfahren zur Untersuchung eines oder mehrerer Phasenobjekte bereitgestellt, wobei ein Gitter aus Elementen verwendet wird, das mit Licht einer Lichtquelle beleuchtet wird, dessen Kohärenzlänge größer als der mittlere Abstand benachbarter Elemente des Gitters ist. Ein Beugungsbild des an dem Gitter gestreuten Beleuchtungslichts wird erzeugt, wobei das eine oder die mehreren Phasenobjekte im Lichtweg zwischen der Lichtquelle und dem Gitter und/oder im Lichtweg des an dem Gitter gestreuten Beleuchtungslichts platziert sind. Zumindest ein Teil des Beugungsbildes wird direkt oder nach Wechselwirkung mit weiteren optischen Komponenten von einem optischen Detektor detektiert und in ein Signal umgewandelt. Das Signal wird weiter analysiert, um daraus Informationen bezüglich des einen oder der mehreren Phasenobjekte zu ermitteln.

Anders als beim beschriebenen Stand der Technik ist keine chemische Bindung zwischen dem Phasenobjekt und dem Gitter erforderlich. Hierdurch kann sich das Phasenobjekt weitestgehend frei auf dem Gitter oder über dem Gitter bewegen, so dass beispielsweise dessen ungehinderte Veränderung, wie z.B. seine ungehinderte Bewegung ermittelt werden kann. Insbesondere ist es mit dem beschriebenen Verfahren möglich, einen Grad der Bedeckung des Gitters durch das eine oder die mehreren Phasenobjekte, eine Anzahl der Phasenobjekte, eine Veränderung des Grads der Bedeckung oder der Anzahl der Phasenobjekte und/oder eine Bewegung, eine Beweglichkeit, einen Typ, eine Form, eine Oberflächenveränderung, eine Formänderung, einen Brechungsindex, eine Volumenänderung, eine auf das eine oder die mehreren Phasenobjekte ausgeübte Kraft, eine von dem einen oder den mehreren Phasenobjekten ausgeübte Kraft, eine Substanz, der das eine oder die mehreren Phasenobjekte ausgesetzt sind, einen physikalischen Reiz, dem das eine oder die mehreren Phasenobjekte ausgesetzt sind, einen Durchmischungsgrad und/oder eine chemische Zusammensetzung des einen oder der mehreren Phasenobjekte in Echtzeit in einer für normale optische Mikroskopietechnik unmöglichen Zeitauflösung und bei einer großen Anzahl von Phasenobjekten und/oder einer hohen Dichte von Phasenobjekten in kurzer Zeit zu ermitteln. Insbesondere ermöglicht das erfindungsgemäße Verfahren einen quantitativen Mobilitätstest für biologische, pharmazeutische und medizinische Fragestellungen.

Darüber hinaus ist es bei dem beschriebenen Verfahren nicht erforderlich, dass die Elemente des Gitters flexibel sind. Während zwar in einigen Ausführungsformen dennoch ein Gitter mit flexiblen Elementen verwendet wird, kann in anderen Ausführungsformen ein Gitter aus starren Elementen verwendet werden. Ein starres Gitter ist im Allgemeinen einfacher herzustellen und zu reinigen.

Unter einem Phasenobjekt wird im Allgemeinen ein dreidimensionales Objekt verstanden, welches die Phasenbeziehung zwischen der auftreffenden und der reflektierten ebenen Lichtwelle zwischen zwei Gitterelementen verändert. Dies erfordert im Allgemeinen, dass das Phasenobjekt bei einer Wellenlänge des eingestrahlten Lichts zumindest teilweise, bevorzugt vollständig transparent ist. Anders als bei einem Amplitudenobjekt ist es jedoch bei einem Phasenobjekt nicht erforderlich, dass das Phasenobjekt Pigmente enthält, die einen Anteil des eingestrahlten Lichts absorbieren. Ein Phasenobjekt weist im Allgemeinen einen Brechungsindex auf, der sich von demjenigen eines das Phasenobjekt umgebenden Mediums unterscheidet. Beispiele für Phasenobjekte sind biologische Zellen, wie z.B. Krebszellen, insbesondere Brustkrebszellen, biologische Gewebe, Kleinsttiere, Fluide, wie beispielsweise Flüssigkeitstropfen, Flüssigkeitsfilme, Gasschichten, etc.

Die beschriebene Platzierung des einen oder der mehreren Phasenobjekte im Lichtweg stellt sicher, dass eine Veränderung an dem Phasenobjekt zu einem veränderten Beugungsbild führt. In vielen Fällen ist das eine Phasenobjekt oder sind die mehreren Phasenobjekte auf dem Gitter angeordnet. Das eine oder die mehreren Phasenobjekte können unmittelbar auf dem Gitter platziert sein und beispielsweise ein oder mehrere Elemente des Gitters berühren. Alternativ kann ein transparentes Medium zwischen dem einen oder den mehreren Phasenobjekten und den Elementen des Gitters und/oder um das eine oder die mehreren Phasenobjekte herum vorgesehen sein, so dass sie das eine oder die mehreren Phasenobjekte nicht in unmittelbarem Kontakt zu den Elementen des Gitters stehen. Beispielsweise kann eine Matrix auf dem Gitter angeordnet sein, wie unten im Detail beschrieben wird, und/oder es kann eine Schutzschicht auf oder unter dem Gitter angeordnet sein, wie ebenfalls unten beschrieben ist. Die Platzierung der Phasenobjekte auf dem Gitter bedeutet im Allgemeinen, dass die Phasenobjekte zumindest teilweise im Lichtweg des auf das Gitter treffenden und/oder von dem Gitter gebeugten Lichts angeordnet sind.

In einigen Ausführungsformen kann das Licht vor und/oder nach dem Auftreffen auf dem Gitter durch ein Umgebungsmedium mit einem ersten Brechungsindex verlaufen, wobei das eine oder die mehreren Phasenobjekte einen zweiten Brechungsindex aufweisen, der von dem ersten Brechungsindex verschieden ist. Es kann beispielsweise vorgesehen sein, dass sich das eine Phasenobjekt oder die mehreren Phasenobjekte in einem Umgebungsmedium, wie beispielsweise Luft, Wasser oder einer wässrigen Lösung befinden, das im Lichtweg zwischen der Lichtquelle und dem Gitter angeordnet ist. Beispielsweise kann das Umgebungsmedium Bereiche auf dem Gitter bedecken, die nicht von dem einen oder den mehreren Phasenobjekten bedeckt sind. Alternativ oder zusätzlich kann sich das Umgebungsmedium auf der dem Gitter abgewandten Seite des einen oder der mehreren Phasenobjekte befinden. Das oder die Phasenobjekte weisen dabei einen anderen Brechungsindex als das Umgebungsmedium auf.

Um das Gitter vor chemischen wie physikalischen Einflüssen zu schützen, kann beispielsweise eine transparente resistente Schutzschicht auf dem Gitter aufgebracht sein, beispielsweise Siliziumoxid. Alternativ kann ein transparentes Substrat verwendet werden, wobei sich das Gitter auf der Rückseite des Substrats befindet. Derartige Ausgestaltungen sind weiter unten näher erläutert.

Durch die Anwesenheit eines Phasenobjekts wird die Phasenlage und u.U. auch der Lichtweg des eingestrahlten Lichts, das an dem Gitter gebeugt wird, verändert, wie dies weiter unten noch im Detail beschrieben werden wird. Beispielsweise wird das ankommende Licht, das durch das Umgebungsmedium verläuft, beim Eintritt in das Phasenobjekt gebrochen, bevor es eines der Gitterelemente erreicht. Nach der Reflektion am Gitterelement wird das Licht beim Übergang aus dem Phasenobjekt heraus in das Umgebungsmedium erneut gebrochen. Somit verlängert sich die Weglänge des Lichts, wenn sich über einem Gitterelement ein Phasenobjekt befindet um einen Gangunterschied, wie er weiter unten noch quantifiziert werden wird. Alternativ kann das Phasenobjekt derart platziert sein, dass es sich nur im Lichtweg zwischen Lichtquelle und Gitter oder nur im Lichtweg zwischen Gitter und optischem Detektor befindet. Durch die unterschiedliche Weglänge ist auch die Phasenlage des an dem Gitterelement reflektierten Lichts eine andere. Die von den Gitterelementen reflektierten Lichtwellen addieren sich phasensensitiv und ergeben in der Überlagerung ein Beugungsbild. Durch die Änderung der Phasenlage (einen Gangunterschied) der an einem der Gitterelemente reflektierten Welle bei Anwesenheit eines Phasenobjekts im Lichtweg vor oder nach dem Gitterelement wird somit das Beugungsbild insgesamt geändert. Selbst wenn nur ein Gitterelement oder nur einige wenige Gitterelemente von einer Veränderung des Phasenobjekts erfasst werden, kann dies dennoch eine substantielle Änderung des Beugungsbildes verursachen. Auch bei identischem Lichtweg, z.B. bei senkrechtem Einfall des Lichts auf ein ebenes Phasenobjekt mit einem von einem umgebenden Medium unterschiedlichen Brechungsindex wird aufgrund der Dispersionsrelation des Lichtes eine Gangunterschied des Lichtstrahls erzielt, welcher wiederum zu einer Modulation des Beugungsbildes führt.

Es kann beispielsweise vorgesehen sein, dass die Phasenobjekte nur einen oder einige der Gitterelemente bedecken. Wenn sich die Phasenobjekte bewegen und beispielsweise ein weiteres Gitterelement bedecken oder ein zuvor bedecktes Gitterelement freigeben, ändert sich die Phasenlage des an diesen Gitterelementen gebeugten Lichts, so dass sich auch das gesamte Beugungsbild ändert. Doch selbst wenn in einem Zeitschritt keine "neuen" Gitterelemente von den Phasenobjekten bedeckt werden und auch keine bisher bedeckten Gitterelemente freigegeben werden, kann sich das Beugungsbild dennoch dadurch ändern, dass sich beispielsweise die Form oder der Brechungsindex eines oder mehrerer der Phasenobjekte über einem oder mehreren der Gitterelemente ändert. Auch hierdurch wird die optische Weglänge des austretenden Lichtes und somit dessen Phasenlage geändert, so dass sich auch das Beugungsbild ändert.

In einigen Ausführungsformen kann das eine Phasenobjekt oder können die mehreren Phasenobjekte alle Gitterelemente bedecken. Beispielsweise kann das Phasenobjekt ein Fluid sein, dass sich über dem Gitter befindet, wie beispielsweise eine Flüssigkeit. Mit dem erfindungsgemäßen Verfahren kann dann beispielsweise eine Oberflächenbewegung der Flüssigkeit oder ein Grad einer Durchmischung mit einer anderen Substanz ermittelt werden, da sich in diesen Fällen die Höhe und/oder Form des Phasenobjekts über den Gitterpunkten bzw. der Brechungsindex des Phasenobjekts zeitlich ändert.

Mit dem erfindungsgemäßen Verfahren ist es beispielsweise möglich, die Aktivität lediglich eines einzigen Phasenobjekts, beispielsweise einer einzigen Zelle, mit hoher Auflösung zu untersuchen. Hierzu kann auf dem Gitter ein einziges Phasenobjekt angeordnet sein. Alternativ können auf dem Gitter in einigen Ausführungsformen mehr als drei, insbesondere mehr als 50 und bevorzugt mehr als 100 Phasenobjekte, insbesondere biologische Zellen, angeordnet sein. Auf diese Weise kann eine große Population von Phasenobjekten gleichzeitig untersucht werden, um beispielsweise einen Bedeckungsgrad des Gitters durch die Phasenobjekte, eine Anzahl der Phasenobjekte, eine Veränderung des Grads der Bedeckung oder der Anzahl der Phasenobjekte, eine Beweglichkeit, eine Formänderung, eine Oberflächenänderung o.ä. der Phasenobjekte zu ermitteln. Auf diese Weise können statistisch zuverlässigere Ergebnisse bereitgestellt werden. Da die Untersuchung der Phasenobjekte durch Beugung, d.h. im Fourier-Raum, erfolgt, ist mit der Untersuchung einer großen Anzahl von Phasenobjekten auch kein Mehraufwand bei der Messung verbunden. Vielmehr wird hierdurch das Spektrum der durch die Gesamtheit der Phasenobjekte verursachten Bewegung analysiert, so dass man z.B. bei der Beobachtung von Zellen als Phasenobjekte ein aussagekräftiges Signal über die Motilität und den Zustand des Zytoskeletts erhält. Das erfindungsgemäße Verfahren erlaubt sogar die Beobachtung in Echtzeit, so dass beispielsweise die Wirkung von Pharmaka auf die Zellen im Detail untersucht werden kann.

Auch die Größe des Beobachtungsfeldes kann auf einfache Weise für eine große Anzahl von Phasenobjekten angepasst werden, indem das Gitter hinreichend groß ausgeführt wird und der Lichtstrahl vor dem Beleuchten des Gitters falls nötig mittels einer Optik aufgeweitet wird.

Der optische Detektor wandelt das detektierte Beugungsbild oder den detektierten Teil des Beugungsbildes in ein Signal um, indem er das Signal basierend auf dem detektierten Beugungsbild oder dem detektierten Teil des Beugungsbildes erzeugt. Das Signal kann beispielsweise eine räumliche Verteilung des gestreuten Beleuchtungslichts angeben. Alternativ oder zusätzlich kann das Signal die Intensität des gestreuten Beleuchtungslichts in einem oder mehreren Teilen des Beugungsbildes angeben, beispielsweise an einem Haupt- oder Nebenmaximum des Beugungsbildes, wie weiter unten erläutert werden wird. Das Signal kann beispielsweise ein elektrisches oder ein elektromagnetisches Signal sein, beispielsweise ein Lichtsignal oder ein Funksignal. Der optische Detektor kann dazu eingerichtet sein, dass Signal über eine drahtgebundene oder eine drahtlose Verbindung, wie beispielsweise ein elektrisch leitfähiges Kabel, ein Glasfaserkabel oder eine Funkverbindung, an die Auswerteschaltung zu übertragen. Alternativ oder zusätzlich kann der optische Detektor dazu eingerichtet sein, das Signal zu speichern, beispielsweise in einem nichtflüchtigen Speicher wie z.B. einer Festplatte oder einem Flash-Speicher. Das Signal kann ein analoges oder ein digitales Signal sein.

In einer bevorzugten Ausführungsform detektiert der optische Detektor eine zeitliche Abfolge von Beugungsbildern oder Teilen von Beugungsbildern und wandelt sie in eine Abfolge von Signalen um, und die Abfolge der Signale wird weiter analysiert, um daraus die Informationen bezüglich des einen oder der mehreren Phasenobjekte zu ermitteln. Unter einer zeitlichen Abfolge wird hier eine Folge von zumindest zwei zeitlich nacheinander detektierten Beugungsbildern oder Teilen von Beugungsbildern verstanden. Zwei Beugungsbilder oder Teile von Beugungsbildern sind beispielsweise ausreichend, um eine Veränderung des einen oder der mehreren Phasenobjekte zwischen den Zeitpunkten, an denen die Beugungsbilder oder Teile von Beugungsbildern aufgenommen wurden, feststellen zu können. Für eine bessere Zeitauflösung und zur Bestimmung der Reaktion des Phasenobjekts auf chemische, biologische oder physikalische Einwirkungen ist es jedoch im Allgemeinen vorteilhaft, wenn eine zeitliche Abfolge von mehr als zwei Beugungsbildern oder Teilen von Beugungsbildern detektiert und in eine Abfolge von Signalen umgewandelt wird, und die Abfolge der Signale analysiert wird, wie unten noch näher erläutert werden wird.

Es ist bevorzugt, dass eine zeitliche Abfolge des Beugungsbildes oder der Teile von Beugungsbildern bei einer gleichen räumlichen Anordnung aus dem Gitter und dem optischen Detektor detektiert wird, so dass die detektierten Beugungsbilder oder Teile des Beugungsbilds einfacher miteinander verglichen werden können. Durch die Analyse der Abfolge der Signale können beispielsweise Veränderungen des einen oder der mehreren Phasenobjekte ermittelt werden, wie z.B. eine Formänderung, eine Oberflächenveränderung, eine Bewegung, etc.

Die zu ermittelnde Veränderung kann dabei eine beliebige Veränderung des einen oder der mehreren Phasenobjekte sein, die einen Brechungsindex zumindest lokal an einer Position in dem Lichtweg von der Lichtquelle zum Gitter und/oder vom Gitter zum optischen Detektor verändert und/oder eine Grenzfläche zwischen zwei optischen Medien mit unterschiedlichen Brechungsindizes ändert. Beispielsweise kann die Veränderung eine Veränderung innerhalb des Phasenobjekts sein, wie beispielsweise eine Diffusionsbewegung innerhalb einer Flüssigkeit. Durch die Diffusion kann sich der Brechungsindex lokal verändern, wodurch sich die Phasenlage des austretenden Lichts und somit das Beugungsbild ändern kann. Durch die hohe Sensitivität des erfindungsgemäßen Verfahrens können derartige Diffusions-oder Mischvorgänge auch bei Verwendung sehr geringer Mengen zuverlässig beobachtet werden. Somit wird Material gespart, potentiell gefährlicher Abfall vermieden, und es werden Analysekosten gesenkt. Die Veränderung kann in anderen Fällen z.B. eine Verschiebung einer Membran zwischen zwei oder mehr Fluidreservoirs innerhalb des Phasenobjekts umfassen, wie beispielsweise eine Membran zwischen Zellbestandteilen. Auch eine Veränderung der Oberfläche, des Volumens, der Ausrichtung oder der Position eines Phasenobjekts kann mit Hilfe des erfindungsgemäßen Verfahrens bestimmt werden, wie unten noch näher erläutert werden wird. Weitere Beispiele von Veränderungen von Phasenobjekten, die mit dem erfindungsgemäßen Verfahren detektiert werden können, werden unten beschrieben.

In einer bevorzugten Ausführungsform werden zumindest zwei Teile des Beugungsbildes von zumindest zwei räumlich voneinander getrennten optischen Detektoren gleichzeitig detektiert und in Signale umgewandelt, wobei die Signale weiter analysiert werden, um daraus die Informationen bezüglich des einen oder der mehreren Phasenobjekte zu ermitteln. Die optischen Detektoren können beispielsweise derart angeordnet sein, dass sie unterschiedliche Haupt-und/oder Nebenmaxima des Beugungsbildes detektieren. In dieser Ausführungsform können die Signale beispielsweise die Intensitäten der unterschiedlichen Haupt- und/oder Nebenmaxima des Beugungsbildes angeben.

In einigen Ausführungsformen wird ein Teil des Beugungsbildes, der beispielsweise ein oder mehrere Hauptmaxima und/oder ein oder mehrere Nebenmaxima umfasst, detektiert. Ein Hauptmaximum des Beugungsbildes entsteht bei Detektion in Reflexion an dem Gitter an Raumpunkten, an denen die an den Gitterelementen reflektierten Lichtstrahlen positiv interferieren. Bei Detektion in Transmission durch das Gitter entsteht ein Hauptmaximum an Raumpunkten, an denen die zwischen den Gitterelementen hindurch verlaufenden Lichtstrahlen positiv interferieren. Nebenmaxima entstehen an Raumpunkten, an denen nur einige dieser Lichtstrahlen positiv miteinander interferieren. Es kann vorgesehen sein, dass beispielsweise die Intensität in einem räumlich feststehenden Teil des Beugungsbildes detektiert wird, der z.B. nur ein Hauptmaximum umfasst.

In einer bevorzugten Ausführungsform wird zumindest die Intensität eines Hauptmaximums des Beugungsbildes von dem optischen Detektor detektiert. Hierzu kann beispielsweise ein Photodetektor als optischer Detektor verwendet werden. Zusätzlich können die Intensitäten weiterer Hauptmaxima und/oder die Intensitäten von Nebenmaxima, z.B. mit dem gleichen optischen Detektor oder einem oder mehreren weiteren optischen Detektoren erfasst werden.

In einer bevorzugten Ausführungsform ist zwischen dem Gitter und dem einen oder den mehreren Phasenobjekten eine transparente Schutzschicht angeordnet, wie sie oben beschrieben worden ist. Die Schutzschicht kann beispielsweise dazu dienen, eine verbesserte Kompatibilität zwischen dem Phasenobjekt und den Gitterelementen zu erzielen oder auch die Oberfläche des Gitters gegen chemische und mechanische Einflüsse zu schützen. Die Schicht kann beispielsweise nur wenige Nanometer bis einige Mikrometer dick sein. Sie sollte bei der Wellenlänge des Beleuchtungslichts transparent sein. Die Schutzschicht kann beispielsweise eine hinreichende mechanische Stabilität aufweisen, um als Substrat für das Gitter zu fungieren.

In einigen bevorzugten Ausführungsformen können das eine oder die mehreren Phasenobjekte in einem Behälter angeordnet sein, der im Lichtweg zwischen der Lichtquelle und dem Gitter und/oder im Lichtweg des an dem Gitter gestreuten Beleuchtungslichts angeordnet oder anordenbar ist, wobei sich das Gitter außerhalb des Behälters befindet. Auf diese Weise wird ein direkter Kontakt zwischen dem Gitter und den Phasenobjekten vermieden, so dass das Gitter geschützt wird und die Phasenobjekte nicht durch das Gitter beeinflusst werden.

Gemäß einer bevorzugten Ausführungsform sind die Phasenobjekte biologische Zellen oder Kleinsttiere. Dies ist insbesondere bei der eingangs erwähnten Anwendung im medizinischen Bereich der Fall. Insbesondere kann das beschriebene Verfahren dazu dienen, eine Zellaktivität, wie beispielsweise eine Zellmotilität, biologischer Zellen zu ermitteln.

In einer bevorzugten Ausführungsform wird aufgrund des Signals eine Veränderung des einen oder der mehreren Phasenobjekte ermittelt. In Ausführungsformen, in denen der optische Detektor eine zeitliche Abfolge von Beugungsbildern oder Teilen von Beugungsbildern detektiert, kann die Veränderung des einen oder der mehreren Phasenobjekte insbesondere aufgrund der Abfolge der Signale ermittelt werden.

Gemäß einer besonders bevorzugten Ausführungsform werden das eine oder die mehreren Phasenobjekte ferner einer Substanz und/oder einem physikalischen Reiz ausgesetzt. Auf diese Weise kann beispielsweise die Auswirkung eines Medikamentes auf Krebszellen untersucht werden, indem z.B. eine Veränderung der Zellaktivität ermittelt wird. Ferner erlaubt diese Ausführungsform eine Untersuchung der Pharmakokinetik von Medikamenten in Echtzeit. Der physikalische Reiz kann beispielsweise eine Beleuchtung mit einem Anregungslicht, eine Erwärmung, ein elektromagnetisches Feld, einer Röntgenbestrahlung, eine radioaktive Bestrahlung, eine mechanische Einwirkung mit einem Gegenstand o.ä. umfassen.

Gemäß einer bevorzugten Ausführungsform wird aufgrund des Signals eine Oberflächen- und/oder Volumenänderung des einen oder der mehreren Phasenobjekte, ein Prozess innerhalb des einen oder der mehreren Phasenobjekte, eine Änderung der Dichte des einen oder der mehreren Phasenobjekte und/oder eine Bewegung des einen oder der mehreren Phasenobjekte relativ zu dem Gitter ermittelt.

Gemäß einer bevorzugten Ausführungsform wird aufgrund des Signals ein Grad einer Durchmischung von zumindest zwei Phasenobjekten ermittelt. Die Phasenobjekte können beispielsweise am Anfang der Untersuchung in verschiedenen Raumbereichen angeordnet sein und sich im Zeitverlauf vermischen, beispielsweise aufgrund einer Diffusion oder aufgrund einer mechanischen Einwirkung, z.B. durch Rühren.

Gemäß einer bevorzugten Ausführungsform wird aufgrund des Signals ein Grad einer Bedeckung des Gitters durch das eine oder die mehreren Phasenobjekte, eine Anzahl der Phasenobjekte und/oder eine Veränderung des Grads der Bedeckung oder der Anzahl der Phasenobjekte ermittelt. Insbesondere kann aufgrund des Signals eine zeitliche Veränderung des Grads der Bedeckung des Gitters durch das eine oder die mehreren Phasenobjekte oder der Anzahl der Phasenobjekte ermittelt werden. In Ausführungsformen, in denen die Phasenobjekte biologische Zellen sind, kann beispielsweise ein Wachstum einer Zellpopulation ermittelt werden. Auf ähnliche Weise kann eine Veränderung, insbesondere ein Wachstum einer Population von Kleinstlebewesen wie z.B. Viren, Pilzen oder Bakterien ermittelt werden, die in alternativen Ausführungsformen die Phasenobjekte bilden. Es kann ferner vorgesehen sein, dass die Phasenobjekte einer Substanz und/oder einem physikalischen Reiz ausgesetzt werden, wie an anderer Stelle beschrieben ist. Hierdurch kann der Einfluss der Substanz oder des physikalischen Reizes auf die Veränderung der Population bestimmt werden. Dies ermöglicht beispielsweise eine qualitative oder quantitative Charakterisierung der Wirksamkeit von Antibiotika, Fungiziden, etc.

Gemäß einer bevorzugten Ausführungsform ist das Gitter auf einem Substrat, insbesondere einem starren Substrat angeordnet. Dies ermöglicht eine einfachere Gitterherstellung mit Standardlithographieverfahren wie sie beispielsweise in der Halbleiterindustrie verwendet werden. Außerdem ist somit eine höhere mechanische Stabilität gewährleistet.

In anderen Ausführungsformen kann allerdings vorgesehen sein, dass das Substrat flexibel ist. Auf diese Weise kann das Beugungsbild auf einfache Weise verändert werden, indem das Substrat verbogen wird. Hierdurch kann das Substrat beispielsweise auf verschiedene verwendete Phasenobjekte eingestellt werden.

Gemäß einer bevorzugten Ausführungsform wird das Beugungsbild oder der Teil des Beugungsbildes zumindest teilweise in Reflexion an dem Gitter detektiert. In dieser Ausführungsform entsteht das Beugungsbild durch die Interferenz der an dem Gitter reflektierten Lichtstrahlen. Hierzu sind die Elemente des Gitters reflektierend ausgebildet. Alternativ oder zusätzlich kann das Beugungsbild oder der Teil des Beugungsbildes zumindest teilweise in Transmission durch das Gitter detektiert werden. In diesen Ausführungsformen entsteht das Beugungsbild durch die Interferenz der zwischen den Gitterelementen durch das Gitter hindurch tretenden Lichtstrahlen. Es kann auch vorgesehen sein, das Beugungsbild oder einen Teil davon sowohl in Reflexion als auch in Transmission zu detektieren. Hierzu kann ein einziger optischer Detektor oder bevorzugt eine Mehrzahl von optischen Detektoren verwendet werden, beispielsweise ein oder mehrere optische Detektoren zur Detektion in Reflexion und ein oder mehrere optische Detektoren zur Detektion in Transmission.

In einer bevorzugten Ausführungsform ist das Gitter auf einem transparenten Substrat, wie beispielsweise einem Glassubstrat, angeordnet. Dies ermöglicht zum einen eine optische Untersuchung der Phasenobjekte mit anderen Verfahren, bei denen durch die Phasenobjekte hindurch beleuchtet wird, zum Beispiel mit einem optischen Transmissionsmikroskop. Zum anderen erlaubt ein transparentes Substrat aber auch eine Detektion des Beugungsbildes oder eines Teils davon in Transmission durch das Substrat alternativ oder zusätzlich zu einer Detektion des Beugungsbildes oder eines Teils davon in Reflektion an dem Gitter. In alternativen Ausführungsformen besteht das Substrat aus Silizium, Siliziumnitrid oder anderen phasenobjektkompatibel Festkörpern.

In einigen Ausführungsformen weisen die Elemente des Gitters eine Abmessung in einem Bereich von 5 nm bis 200 $\mu$m, insbesondere von 20 nm bis 80 $\mu$m und vorzugsweise von 100 nm bis 10 $\mu$m auf. Derartige Abmessungen sind besonders geeignet, um die Beweglichkeit von biologischen Zellen zuverlässig zu ermitteln.

Ein Zwischenraum zwischen benachbarten Gitterelementen kann leer sein oder kann mit einem Füllmaterial gefüllt sein.

In einigen Ausführungsformen weist das Gitter eine Periode in einem Bereich von 10 nm bis 100 $\mu$m, insbesondere von 40 nm bis 50 $\mu$m und bevorzugt von 200 nm bis 20 $\mu$m auf. Derartige Werte für die Gitterperiode sind beispielsweise für die Bestimmung der Zellmotilität vorteilhaft. Beispielsweise kann die Gitterperiode so gewählt werden, dass das Phasenobjekt zumindest ein Gitterelement wenigstens teilweise bedeckt. Hierzu ist es vorteilhaft, wenn die Gitterperiode geringer als eine minimale Abmessung, insbesondere eine minimale laterale Abmessung des Phasenobjekts, ist, wie beispielsweise höchstens ein Fünftel der minimalen Abmessung des Phasenobjekts.

Gemäß einer bevorzugten Ausführungsform sind die Elemente des Gitters periodisch angeordnet. Allerdings kann in anderen Ausführungsformen vorgesehen sein, dass das Gitter quasiperiodisch ist.

Gemäß einer bevorzugten Ausführungsform ist das Gitter ein zweidimensionales Gitter. Dies ist gegenüber anderen Ausführungsformen, in welchen das Gitter ein eindimensionales Gitter ist, bevorzugt, da Informationen bezüglich des Phasenobjektes in zwei Dimensionen ermittelt werden.

In einer bevorzugten Ausführungsform umfasst die besagte zeitliche Abfolge zumindest drei, insbesondere zumindest 20 und bevorzugt zumindest 100 zeitlich nacheinander detektierte Beugungsbilder oder Teile von Beugungsbildern. Dabei ermöglicht eine höhere Anzahl zeitlich nacheinander detektierter Beugungsbilder im Allgemeinen eine größere Genauigkeit bei der Ermittlung der Information bezüglich des einen oder der mehreren Phasenobjekte.

In einer bevorzugten Ausführungsform wird durch eine Verarbeitung der Abfolge der Signale eine Korrelation, insbesondere eine Standardabweichung und/oder Fourieranalyse der zeitlichen Abfolge der detektierten Beugungsbilder oder Teile der Beugungsbilder ermittelt. Also bevorzugte Information der Beugungsbilder dient die Lichtintensität. Hierdurch kann auf einfache Weise eine Veränderung der Phasenobjekte zwischen zwei nacheinander aufgenommenen Beugungsbildern oder Beugungsbildteilen festgestellt werden. Hierdurch lassen sich beispielsweise quantifizierbare Informationen beispielsweise über die Veränderung des einen oder der mehreren Phasenobjekte ermitteln. Unabhängig davon, ob eine Fourieranalyse der zeitlichen Abfolge durchgeführt wird, kann an jedem einzelnen Beugungsbild oder Beugungsbildteil der zeitlichen Abfolge eine Fourieranalyse durchgeführt werden.

Durch das erfindungsgemäße Verfahren wird eine Untersuchung von Phasenobjekten mit sehr hoher Zeitauflösung ermöglicht, so dass beispielsweise auch eine sehr große Anzahl wie auch schnelle Veränderungsprozesse der Phasenobjekte untersucht werden können. Ein weiterer Vorteil eines kurzen zeitlichen Abstands zwischen den detektierten Beugungsbildern oder Teilen von Beugungsbildern ist es, dass eine langsame Veränderung des Beleuchtungslichts, wie z.B. ein Laserdrift, die Ergebnisse des Verfahrens nicht signifikant beeinträchtigt. Im Allgemeinen sind aber Beobachtungen von Veränderungen der Phasenobjekte beispielsweise vom Nanosekunden- bis zum Minutenbereich bzw. Stunden- und Tagebereich möglich.

In einigen Ausführungsformen wird das Licht intermittierend, z.B. pulsweise eingestrahlt. Auf diese Weise werden die Phasenobjekte nicht dauerhaft beleuchtet, um eine Beeinflussung des Phasenobjekts, wie beispielsweise einer Zelle, durch das Licht möglichst zu reduzieren.

Gemäß einer bevorzugten Ausführungsform wird anhand des Signals eine Charakteristik des einen oder der mehreren Phasenobjekte ermittelt, und die ermittelte Charakteristik wird mit Einträgen in einer Datenbank und/oder einem theoretischen Modell verglichen, um die Informationen bezüglich des einen oder der mehreren Phasenobjekte, insbesondere einen Grad der Bedeckung des Gitters durch das eine oder die mehreren Phasenobjekte, eine Anzahl der Phasenobjekte, eine Veränderung des Grads der Bedeckung oder der Anzahl der Phasenobjekte und/oder eine Bewegung, eine Beweglichkeit, einen Typ, eine Form, eine Oberflächenveränderung, eine Formänderung, einen Brechungsindex, eine Dichteänderung, eine Volumenänderung, eine auf das eine oder die mehreren Phasenobjekte ausgeübte Kraft, eine von dem einen oder den mehreren Phasenobjekten ausgeübte Kraft, eine Substanz, der das eine oder die mehreren Phasenobjekte ausgesetzt sind, einen physikalischen Reiz, dem das eine oder die mehreren Phasenobjekte ausgesetzt sind, eine Durchmischungsgrad und/oder eine chemische Zusammensetzung des einen oder der mehreren Phasenobjekte zu ermitteln.

Beispielsweise kann in der Datenbank gespeichert sein, dass eine bestimmte anhand des Signals oder der Abfolge der Signale ermittelte Charakteristik "gesunden", "kranken" oder "verdächtigen" Zellen eines bestimmten Zelltyps zugeordnet ist. Auf diese Weise kann automatisiert eine Typisierung (Zelldiagnostik) durchgeführt werden, und es können Warnhinweise gegeben werden, wenn sich die Phasenobjekte auf ungewöhnliche oder unerwartete Weise verändern, z.B. bewegen. Beispielsweise kann ein Hinweis gegeben werden, wenn eine Zellaktivität wie beispielsweise eine Zellmotilität oder ein Zellwachstum zu hoch oder zu gering ist, wobei die Typisierung "zu hoch" und "zu gering" in der Datenbank - z.B. in Abhängigkeit des Zelltyps - gespeichert ist. Die zum Vergleich mit den Einträgen der Datenbank verwendete Charakteristik kann beispielsweise Fourier-Koeffizienten und/oder Intensitäten umfassen, die sich bei der Fourieranalyse der zeitlichen Abfolge von detektierten Beugungsbildern oder detektierten Teilen der Beugungsbilder ergeben, die anhand der Abfolge der Signale durchgeführt wird, oder eine andere aus der Abfolge ermittelte Charakteristik.

In einigen Ausführungsformen ist auf dem Gitter eine dreidimensionale Matrix angeordnet, in oder auf welcher das eine oder die mehreren Phasenobjekte angeordnet sind, um eine dreidimensionale Veränderung, z.B. eine dreidimensionale Bewegung, Beweglichkeit oder Formänderung des einen oder der mehreren Phasenobjekte zu ermitteln. Beispielsweise kann das Gitter mit der Matrix beschichtet sein. Die Matrix kann zumindest teilweise transparent sein, um das Licht hindurchtreten zu lassen. Die Matrix kann ein Gel, beispielsweise ein Polymergel oder Kollagen umfassen. Hiermit können Phasenobjekte, insbesondere lebende Zellen, in oder auf verschieden Umgebungen untersucht werden. Bei bekannter Elastizität der Matrix ermöglicht diese Ausführungsform auch das Ermitteln einer von dem einen oder den mehreren Phasenobjekten ausgeübten Kraft.

In einem weiteren Aspekt wird eine Vorrichtung zur Untersuchung eines oder mehrerer Phasenobjekte bereitgestellt. Die Vorrichtung umfasst eine Aufnahme zum Aufnehmen eines Sensors mit einem Gitter aus Elementen. Ferner umfasst die Vorrichtung eine Lichtquelle, die so angeordnet oder anordenbar ist, dass sie einen in oder auf der Aufnahme angeordneten Sensor derart beleuchten kann, dass durch an dem Gitter gestreutes Licht der Lichtquelle ein Beugungsbild erzeugt wird und ein oder mehrere zu untersuchende Phasenobjekte im Lichtweg zwischen der Lichtquelle und dem Gitter und/oder im Lichtweg des an dem Gitter gestreuten Beleuchtungslichts angeordnet sind, sowie einen optischen Detektor, der dazu eingerichtet ist, zumindest einen Teil des Beugungsbildes direkt oder nach Wechselwirkung mit weiteren optischen Komponenten zu detektieren und in ein Signal umzuwandeln. Darüber hinaus umfasst die Vorrichtung eine Auswerteschaltung, die an den optischen Detektor gekoppelt ist und das Signal empfängt, und die dazu ausgelegt ist, aus dem Signal Informationen bezüglich des einen oder der mehreren Phasenobjekte zu ermitteln.

Die Auswerteschaltung kann z.B. eine Analog- oder Digitalschaltung umfassen. Beispielsweise kann die Auswerteschaltung einen Prozessor umfassen, der dazu programmiert ist, die angegebenen Schritte durchzuführen.

In einer bevorzugten Ausführungsform umfasst der optische Detektor einen Bildsensor und/oder einen Photodetektor. Der optische Detektor kann z.B. eine oder mehrere Photodioden und/oder CCD-Kameras umfassen. Der optische Detektor kann das Beugungsbild oder den Teil des Beugungsbildes in ein elektrisches Signal umzuwandeln, wie beispielsweise ein Spannungs- oder Stromsignal oder einen Pixelwert, welcher proportional zur Intensität ist, und das elektrische Signal der Auswerteschaltung zuzuführen.

In einer bevorzugten Ausführungsform ist der optische Detektor dazu eingerichtet, eine zeitliche Abfolge von Beugungsbildern oder Teilen von Beugungsbildern zu detektieren und in eine Abfolge von Signalen umzuwandeln, wobei die Auswerteschaltung dazu ausgelegt ist, aus der Abfolge der Signale die Informationen bezüglich des einen oder der mehreren Phasenobjekte zu ermitteln.

In einer bevorzugten Ausführungsform umfasst die zeitliche Abfolge zumindest drei, insbesondere zumindest 20 und bevorzugt zumindest 100 zeitlich nacheinander detektierte Beugungsbilder oder detektierte Teile von Beugungsbildern.

Gemäß einer bevorzugten Ausführungsform ist die Auswerteschaltung dazu ausgelegt, durch eine Verarbeitung der Abfolge der Signale eine Korrelation, insbesondere eine Fourieranalyse der Abfolge der detektierten Beugungsbilder oder der detektierten Teile der Beugungsbilder durchzuführen.

In einer bevorzugten Ausführungsform ist der optische Detektor angeordnet, um zumindest die Intensität und/oder die Position eines Hauptmaximums des Beugungsbildes zu detektieren.

Gemäß einer bevorzugten Ausführungsform ist der optische Detektor ein erster optischer Detektor, der dazu eingerichtet ist, einen ersten Teil des Beugungsbildes zu detektieren und in ein erstes Signal umzuwandeln, und die Vorrichtung umfasst einen zweiten optischen Detektor, der von dem ersten optischen Detektor räumlich getrennt ist und dazu eingerichtet ist, einen zweiten Teil des Beugungsbildes zu detektieren und in ein zweites Signal umzuwandeln, wobei die Auswerteschaltung ferner an den zweiten optischen Detektor gekoppelt ist und das zweite Signal empfängt und dazu ausgelegt ist, aus dem ersten und dem zweiten Signal die Informationen bezüglich des einen oder der mehreren Phasenobjekte zu ermitteln.

In einer bevorzugten Ausführungsform ist der optische Detektor angeordnet, um das Beugungsbild oder den Teil des Beugungsbildes zumindest teilweise in Transmission durch das Gitter und/oder zumindest teilweise in Reflektion an dem Gitter zu detektieren.

Gemäß einer bevorzugten Ausführungsform ist die Auswerteschaltung dazu ausgelegt, aufgrund des Signals eine Veränderung des einen oder der mehreren Phasenobjekte zu ermitteln.

In einer bevorzugten Ausführungsform ist die Auswerteschaltung dazu ausgelegt, aufgrund des Signalseine Oberflächen- und/oder Volumenänderung des einen oder der mehreren Phasenobjekte, einen Prozess innerhalb des einen oder der mehreren Phasenobjekte, eine Änderung der Dichte des einen oder der mehreren Phasenobjekte und/oder eine Bewegung des einen oder der mehreren Phasenobjekte relativ zu dem Gitter zu ermitteln.

Gemäß einer bevorzugten Ausführungsform ist die Auswerteschaltung dazu ausgelegt, aufgrund des Signals einen Grad einer Durchmischung von zumindest zwei Phasenobjekten zu ermitteln.

Gemäß einer bevorzugten Ausführungsform ist die Auswerteschaltung dazu ausgelegt, aufgrund des Signals einen Grad einer Bedeckung des Gitters durch das eine oder die mehreren Phasenobjekte, eine Anzahl der Phasenobjekte und/oder eine Veränderung des Grads der Bedeckung oder der Anzahl der Phasenobjekte zu ermitteln.

In einer bevorzugten Ausführungsform umfasst die Vorrichtung ferner einen Datenspeicher mit einer darin gespeicherten Datenbank oder ist damit koppelbar, und die Auswerteschaltung ist dazu ausgelegt, anhand des Signals eine Charakteristik des einen oder der mehreren Phasenobjekte zu ermitteln, und die ermittelte Charakteristik mit Einträgen in der Datenbank zu vergleichen, um die Informationen bezüglich des einen oder der mehreren Phasenobjekte, insbesondere einen Grad der Bedeckung des Gitters durch das eine oder die mehreren Phasenobjekte, eine Anzahl der Phasenobjekte, eine Veränderung des Grads der Bedeckung oder der Anzahl der Phasenobjekte und/oder eine Bewegung, eine Beweglichkeit, einen Typ, eine Form, eine Oberflächenveränderung, eine Formänderung, einen Brechungsindex, eine Dichteänderung, eine Volumenänderung, eine auf das eine oder die mehreren Phasenobjekte ausgeübte Kraft, eine von dem einen oder den mehreren Phasenobjekten ausgeübte Kraft, eine Substanz, der das eine oder die mehreren Phasenobjekte ausgesetzt sind, einen physikalischen Reiz, dem das eine oder die mehreren Phasenobjekte ausgesetzt sind, einen Durchmischungsgrad und/oder eine chemische Zusammensetzung des einen oder der mehreren Phasenobjekte zu ermitteln.

Die Vorrichtung kann in einigen Ausführungsformen eine Anzeige oder eine Schnittstelle zur Ausgabe der ermittelten Informationen bezüglich des einen oder der mehreren Phasenobjekte umfassen.

In einer bevorzugten Ausführungsform umfasst die genannte optische Komponente eine Fourier-Optik, die angeordnet ist, um das Beugungsbild in ein reelles Bild einer Anordnung des einen oder der mehreren Phasenobjekte umzuwandeln. Dabei wird vorgeschlagen, das Fraunhofer-Beugungsbild durch Wechselwirkung mit einer Optik, beispielsweise einer Linse, aus dem Fourierraum in den Orts- bzw. Ursprungsraum zu transformieren, um wieder ein "normales" Bild der Anordnung der Phasenobjekte zu erhalten. Bei der Optik kann es sich beispielsweise um eine Sammellinse handeln, die als sog. Fourier-Optik eingesetzt wird. Das elektrische Feld des in der bildseitigen Brennebene liegenden Beugungsbildes entspricht dabei der räumlichen Fouriertransformierten des elektrischen Feldes in der objektseitigen Brennebene. Die Verwendung einer derartigen Fourier-Optik gestattet es, ein vergrößertes Realbild zu erzeugen, ohne dass ein Mikroskop verwendet werden müsste, und stellt somit eine kostengünstige Alternative für einen herkömmlichen Aufbau mit Mikroskop dar. Ein weiterer Vorteil dieser Weiterentwicklung besteht darin, dass eine Vorrichtung, die in einem Betriebsmodus im Fourierraum arbeitet (beispielsweise um Intensitäten einzelner Hauptmaxima zu messen), schnell und einfach umgerüstet werden kann, um eine "realoptische" Abbildung der Anordnung der Phasenobjekte zu erhalten. Auf diese Weise erhält man die Vorzüge beider Detektionsmethoden in ein und derselben Vorrichtung mit minimalem zusätzlichem apparativem Aufwand gegenüber einer rein auf Beugungsmusterdetektion basierten Vorrichtung.

In einer bevorzugten Ausführungsform enthält die Vorrichtung ferner einen in oder auf die Aufnahme aufgenommenen Sensor mit einem Gitter aus Elementen.

Dabei ist besonders bevorzugt, dass auf dem Gitter des Sensors eine Schutzschicht angeordnet ist, um darauf das eine oder die mehreren Phasenobjekte anzuordnen

In einer bevorzugten Ausführungsform umfasst die Vorrichtung ferner einen Behälter zur Aufnahme des einen oder der mehreren Phasenobjekte, wobei der Behälter im Lichtweg zwischen der Lichtquelle und dem Gitter und/oder im Lichtweg des an dem Gitter gestreuten Beleuchtungslichts angeordnet oder anordenbar ist, wobei sich der in oder auf der Aufnahme aufgenommene Sensor mit dem Gitter außerhalb des Behälters befindet.


KURZBESCHREIBUNG DER FIGUREN

Weitere Vorteile und Merkmale der Erfindung ergeben sich aus der folgenden Beschreibung, in der die Erfindung anhand von Ausführungsbeispielen unter Bezugnahme auf die beigefügten Zeichnungen erläutert wird. Darin zeigen

Fig. 1 ein auf einem Substrat befindliches Gitter in einer Profilansicht und einer Draufsicht,

Fig. 2 ein anderes auf einem Substrat befindliches Gitter in einer Profilansicht und einer Draufsicht,

Fig. 3 eine schematische Darstellung der Beugung an einem Gitter, das sich in einem Durchflussbehälter befindet,

Fig. 4 eine schematische Darstellung der Beugung an einem Gitter mit darauf platzierten Zellen in einem Behälter,

Fig. 5 eine schematische Darstellung der Beugung an einem Gitter in einer bewegten Flüssigkeit,

Fig. 6a, b eine schematische Darstellung des Verlaufs von Licht bei der Beugung an einem Gitter ohne bzw. mit Phasenobjekt,

Fig. 7          einen typischen Strahlenverlauf bei Reflexion an einem Gitterelement und Durchgang durch ein Phasenobjekt,

Fig. 8          eine schematische Darstellung einer erfindungsgemäßen Vorrichtung,

Fig. 9a        eine Hellfeld-Mikroskop-Aufnahme in Transmission eines Gitters,

Fig. 9b        eine Mikroskop-Aufnahme des Gitters der Fig. 9a in Reflexion,

Fig. 10a       eine Hellfeld-Mikroskop-Aufnahme in Transmission eines Gitters mit darauf platzierter Zelle,

Fig. 10b       eine Mikroskop-Aufnahme des Gitters der Fig. 10a in Reflexion,

Fig. 11a       ein Photo einer erfindungsgemäßen Vorrichtung,

Fig. 11b       eine Detailansicht einer Aufnahme für einen Sensor,

Fig. 12        einen Verlauf eines Detektionssignals in der Zeitdomäne,

Fig. 13        einen Verlauf des detektierten Signals in der Frequenzdomäne,

Fig. 14        einen zeitlichen Verlauf der Fourier-Koeffizienten bei Zugabe eines Wirkstoffs,

Fig. 15a-c     zu den Fig. 3-5 ähnliche Ausführungsformen, bei denen sich das Gitter außerhalb des Behälters befindet,

Fig. 16        die normierte Intensität eines Beugungsmaximums als Funktion der Zeit mit und ohne Bakterienpopulation,

Fig. 17        Messpunkte eines Signals, das der Differenz der Intensitäten ohne und mit Bakterien entspricht, sowie eine zugehörige Fitfunktion,

Fig. 18        den zeitlichen Verlauf der Fluktuationen in der Intensität, und

Fig. 19        in Spalte a) optische Mikroskopbilder einer Probe zum Zeitpunkt 0, in Spalte b) optische Mikroskopbilder zu späteren Zeitpunkten, in Spalte c) die normierte Intensität des Beugungsmaximums als Funktion der Zeit und in Spalte d) die Mittelwerte der Standardabweichung der Intensität als Funktion der Zeit.

## BESCHREIBUNG DER BEVORZUGTEN AUSFÜHRUNGSFORMEN

Im Folgenden wird das erfindungsgemäße Verfahren beispielhaft unter Bezugnahme auf die beigefügten Zeichnungen beschrieben. Dabei werden gleiche Bezugszahlen verwendet, um ähnliche Elemente zu bezeichnen.

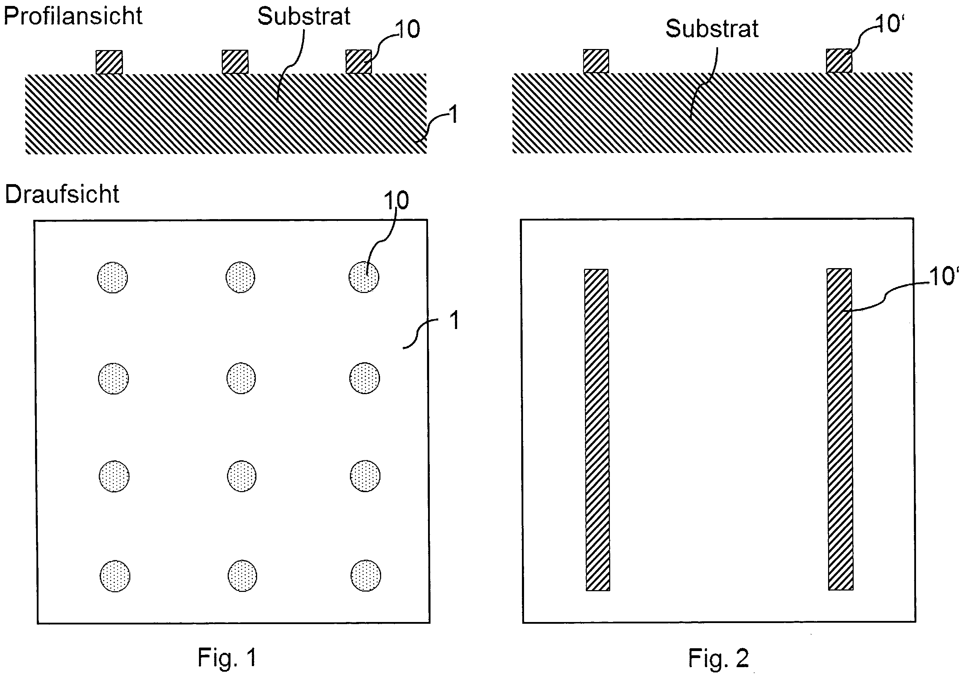

Fig. 1 zeigt ein Gitter aus im Wesentlichen zylindrischen Elementen 10, die auf einem Substrat 1 angeordnet sind. Im oberen Bereich der Fig. 1 ist dabei ein Querschnitt durch das Gitter mit dem Substrat 1 zu sehen, während im unteren Bereich der Fig. 1 eine Draufsicht auf das Gitter dargestellt ist. In der in Fig. 1 gezeigten Ausführungsform ist das Gitter ein zweidimensionales periodisches Gitter, wobei die Gitterperiodizität in die zwei Richtungen unterschiedlich ist. Allerdings kann in anderen Ausführungsformen vorgesehen sein, dass das Gitter ein zweidimensionales Gitter mit gleicher Gitterperiodizität in beide Richtungen ist.

Fig. 2 zeigt ein anderes Gitter mit Elementen 10', das bei dem erfindungsgemäßen Verfahren verwendet werden kann. Die Elemente 10' sind längliche, parallel angeordnete Elemente. Auch wenn in Fig. 2 nur zwei derartige Elemente 10' dargestellt sind, wird der Fachmann verstehen, dass weitere Elemente parallel und beispielsweise jeweils in einem gleichen Abstand angeordnet sein können. Bei dem in Fig. 2 dargestellten Gitter handelt es sich somit um ein eindimensionales Gitter.

Die Elemente 10, 10' der Gitter der Fig. 1 und 2 können beispielsweise aus Gold, Aluminium, Titan und/oder Siliziumnitrid sein, während das Substrat 1 beispielsweise aus Siliziumoxid, Glas, Silizium, Siliziumnitrid oder Galliumarsenid ist. In anderen Ausführungsformen kann das Substrat flexibel sein. In einigen Ausführungsformen kann eine weitere Oberflächenbeschichtung auf dem Gitter vorgesehen sein, wie beispielsweise eine Gel-Matrix. Das Gitter und das Substrat 1 bilden zusammen einen Sensor, der in einer erfindungsgemäßen Vorrichtung eingesetzt werden kann, wie weiter unten noch erläutert wird. Zusätzlich kann das Gitter mit einer Schutzschicht oder einer Matrix versehen sein (nicht dargestellt), wie sie eingangs beschrieben worden sind. Im Folgenden wird im Detail beschrieben, wie mit einem derartigen Sensor Informationen bezüglich eines oder mehrerer Phasenobjekte, wie z.B. Veränderungen eines oder mehrerer Phasenobjekte ermittelt werden können.

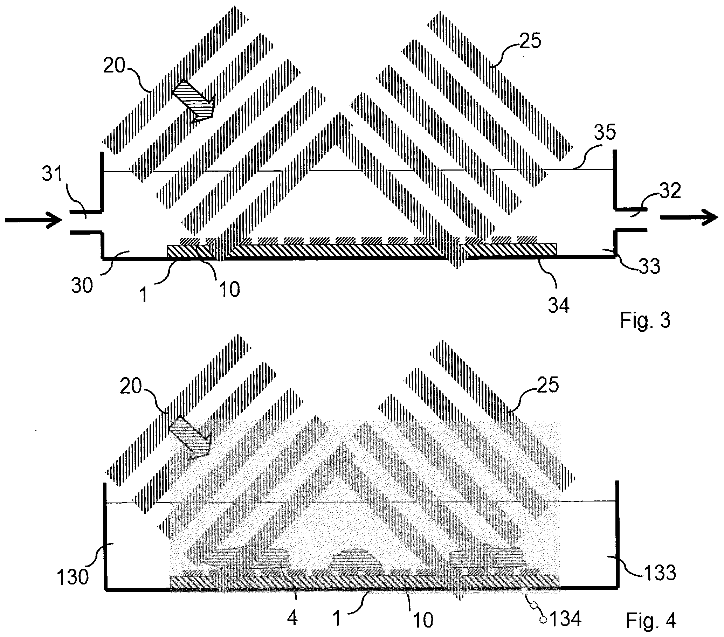

Fig. 3 zeigt eine Aufnahme, die als Durchflussbehälter 30 ausgebildet ist, zum Aufnehmen eines Sensors, wie z.B. des Sensors der Fig. 1 oder 2. Der Durchflussbehälter 30 weist einen Einlass 31 und einen Auslass 32 auf, um ein Fluid 33, wie beispielsweise eine Flüssigkeit, durch den Durchflussbehälter 30 ein- bzw. ausströmen zu lassen. In anderen Ausführungsformen kann das Fluid 33 ein Gas sein. Auf dem Boden 34 des Durchflussbehälters 30 befindet sich ein Sensor, der Elemente 10, die ein Gitter bilden, und ein Substrat 1 aufweist. Das Substrat 1 liegt dabei auf dem Boden 34 des Durchflussbehälters 30 auf, wobei das Gitter auf der dem Boden 34 des Behälters 30 abgewandten Seite des Substrats 1 angeordnet ist.

Einfallendes Licht 20, das beispielsweise von einer kohärenten Lichtquelle, insbesondere einem Laser, stammt, wird schräg auf das Gitter eingestrahlt. Dabei verläuft das Licht 20 in einem Winkel zu der Oberfläche 35 des Fluids 33. Beim Eintritt in das Fluid 33 wird das Licht 20 an der Oberfläche 35 gebrochen (in Fig. 3 nicht im Einzelnen dargestellt). Das Licht 20 trifft nach dem Eintritt in das Fluid 33 auf das Gitter, wobei es an den Elementen 10 des Gitters reflektiert wird. Das gebeugte Licht (in Fig. 3 schematisch mit den Bezugszeichen 25 versehen) tritt durch die Oberfläche 35 wieder aus dem Fluid 33 aus und wird beim Hindurchtreten durch die Oberfläche 35 erneut gebrochen.

Die anhand der Fig. 3 dargestellte Ausführungsform kann beispielsweise dazu verwendet werden, um den Einfluss bzw. das Durchmischen verschiedener Flüssigkeiten innerhalb des Durchflussbehälters 30 zu untersuchen. Beispielsweise wird beim Einfließen des Fluids in den Behälter 30 der Lichtweg, die Phase (aufgrund der Dispersionsrelation) und/oder die Polarisation zwischen der Oberfläche 35 und dem Gitter verändert, so dass sich auch die Phasenlage des austretenden Lichts 25 ändert. Dies beeinflusst das Beugungsbild, das mit einem optischen Detektor zumindest teilweise detektiert werden kann. Beim Durchmischen von Flüssigkeiten, einer chemischen Reaktion oder beim Auflösen eines Feststoffs in dem Fluid 33 verändert sich oft der Brechungsindex des Fluids 33. Hierdurch ändert sich der Brechungswinkel des Lichts 20, 25 an der Oberfläche 35 und auch die Phasenlage des austretenden Lichts 25. Auch hierdurch wird somit das Beugungsbild verändert. Die Umwandlung einer zeitlichen Abfolge von Beugungsbildern oder Teilen von Beugungsbildern des Lichts 25 in eine Abfolge von Signalen und eine anschließende Auswertung der Abfolge der Signale ermöglicht somit eine Bestimmung des Zeitpunkts, ab dem ein Gleichgewicht eingetreten ist. Insbesondere kann bestimmt werden, ab wann eine Gleichgewichtsreaktion abgeschlossen ist oder ab wann eine Flüssigkeit homogen gemischt ist. Das Fluid 33 fungiert somit in dieser Ausführungsform als Phasenobjekt.

Fig. 4 illustriert eine weitere Ausführungsform des erfindungsgemäßen Verfahrens. Dabei ist eine Aufnahme in Form eines Behälters 130 vorgesehen, der mit einem Fluid 133 gefüllt ist. Am Boden 134 des Behälters 130 ist ein Gitter aus Elementen 10 vorgesehen, das auf einem Substrat 1 angeordnet ist. Das Substrat 1 befindet sich auf dem Boden 134 des Behälters 130. Im Vergleich zu der Fig. 3 befinden sich auf dem Gitter der Fig. 4 mehrere Phasenobjekte in der Form biologischer Zellen 4. Durch das Detektieren zeitlich aufeinanderfolgender Beugungsbilder des austretenden Lichts 25 kann eine Veränderung der Anordnung der Zellen 4 auf dem Gitter und somit eine Zellmotilität bestimmt werden. Das Fluid 133 in dem Behälter 130 kann beispielsweise derart ausgewählt sein, dass es einen Brechungsindex aufweist, der sich wesentlich von dem Brechungsindex der Zellen 4 auf dem Gitter unterscheidet. Auf diese Weise bewirkt eine Bewegung der Zellen 4 auf dem Gitter eine deutlichere Veränderung des Beugungsbildes.

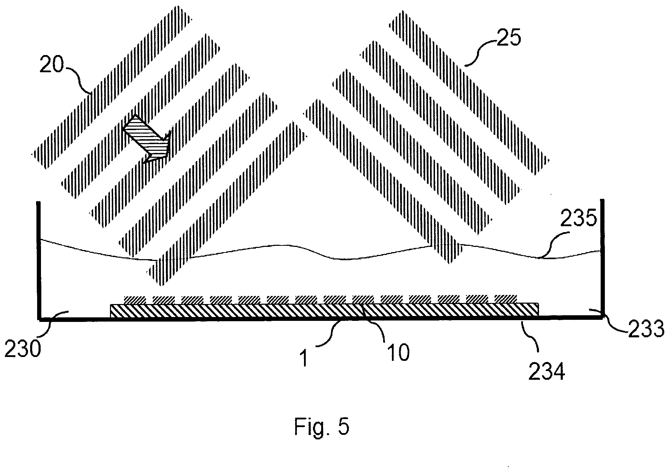

Fig. 5 zeigt eine Aufnahme für einen Sensor in Form eines Behälters 230, die in einer weiteren Ausführungsform verwendet werden kann. In dem Behälter 230, der wie in den Fig. 3 und 4 als Probenkammer ausgebildet ist, befindet sich ein Sensor, der ein Gitter aus Elementen 10 umfasst, das auf einem Substrat 1 angeordnet ist, sowie ein Fluid 233, welches das Gitter bedeckt. Das Substrat 1 liegt auf dem Boden 234 des Behälters 230. Bei dem in Fig. 5 gezeigten Szenario bewegt sich die Oberfläche 235. Durch die zeitliche Veränderung der Oberfläche verändert sich auch lokal die Höhe und die Oberflächenkrümmung des Fluids 233 über den Gitterelementen 10 und somit die Wegstrecke, die das einfallende Licht 20 durch das Fluid 233 zurücklegen muss, bevor es die Gitterelemente 10 erreicht. Somit ändert sich auch die Phasenlage des austretenden Lichts 25. Durch eine Detektion einer zeitlichen Abfolge der Beugungsbilder oder Beugungsbildteile des austretenden Lichts 25 kann somit eine Bewegung der Oberfläche 235 erfasst und beispielsweise visualisiert werden. In dieser Ausführungsform dient das Fluid 233 selbst als Phasenobjekt. Da die Bewegung der Oberfläche beispielsweise durch eine Erschütterung verursacht sein kann, stellt die erfindungsgemäße Vorrichtung somit einen Erschütterungssensor bereit. Dieser kann beispielsweise im Rahmen eines Einbruchsalarms, eines Seismographen o.ä. eingesetzt werden.

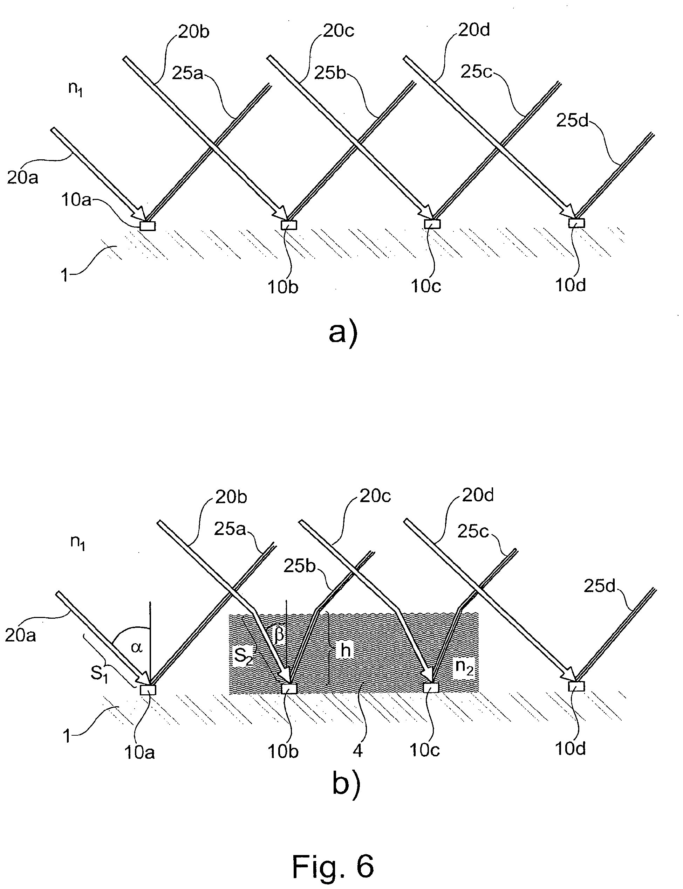

Fig. 6a und 6b illustrieren schematisch die Veränderung der Phasenlage des austretenden Lichts 25 dadurch, dass ein Phasenobjekt auf dem Gitter vorhanden ist. Fig. 6a zeigt dabei den Fall, dass das ankommende Licht 20a-d lediglich an Elementen 10a-d des Gitters reflektiert wird, ohne durch Phasenobjekte zu verlaufen. Das reflektierte Licht 25a-d bildet dabei ein gleichmäßiges Beugungsbild.

Fig. 6b zeigt nun den Fall, dass sich ein Phasenobjekt, wie beispielsweise eine schematisch eingezeichnete Zelle 4, teilweise auf dem Gitter befindet. In dem in Fig. 6b gezeigten Szenario bedeckt die Zelle 4 zwei Gitterelemente 10b,c. Bei der Reflexion an den Gitterelementen 10a,d, die nicht von der Zelle 4 bedeckt sind, ändert sich im Vergleich zu der Fig. 6a nichts. Doch bei den Gitterelementen 10b,c, welche sich unterhalb der Zelle 4 befinden, muss das eintretende Licht 20 zunächst um eine Strecke $s_2$ durch das Material der Zelle 4 verlaufen, bevor es auf die Gitterelemente 10b,c trifft. Beim Eintritt in die Zelle 4 erfährt das Licht 20b,c dabei eine Brechung, die durch den Brechungsindex $n_1$ des Umgebungsmediums und den Brechungsindex $n_2$ der Zelle 4 gegeben ist. Auch nach der Reflexion an den Gitterpunkten 10b,c verläuft das reflektierte Licht 25b,c zunächst durch die Zelle 4, bevor es an deren Oberfläche erneut gebrochen wird.

Beim Vergleich der Weglänge des austretenden Lichts 25a,b an den Gitterelementen 10a und 10b erkennt man, dass durch die Anwesenheit der Zelle 4 über dem Gitterelement 10b der Strahlengang des einfallenden und reflektierten Lichts verändert wird. Durch den um $s_2-s_1$ längeren Weg an dem Gitterelement 10b, über dem sich die Zelle 4 befindet, im Vergleich zu dem Gitterelemente 10a in Fig. 6b oder auch dem Gitterelement 10b in Fig. 6a (d.h. ohne Phasenobjekt), verschiebt sich auch die Phasenlage des reflektierten Lichts 25b. Diese Phasenänderung beeinflusst das Beugungsbild, das durch die austretenden Lichtstrahlen 25a-d gebildet wird. Durch die Detektion des Beugungsbildes oder eines Teils davon kann somit eine Lage der Zelle 4 auf dem Gitter ermittelt werden. Darüber hinaus ermöglicht die Detektion einer zeitlichen Abfolge von Beugungsbildern auch eine Bestimmung einer Veränderung, z.B. einer Bewegung der Zelle.

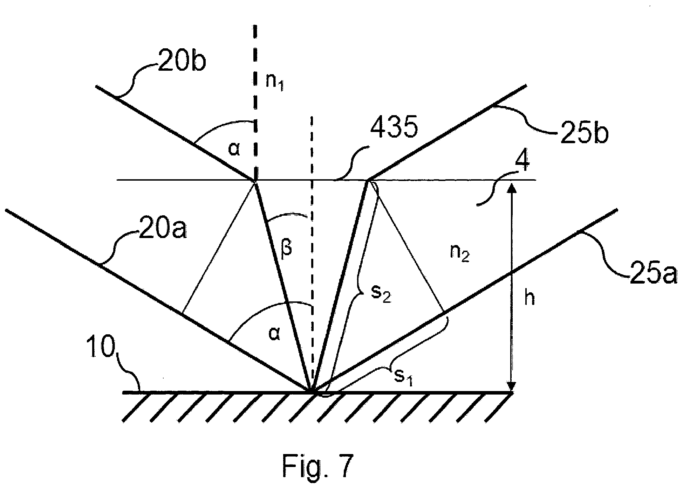

Fig. 7 illustriert den mathematischen Zusammenhang zwischen dem Gangunterschied des reflektierten Lichtstrahls 25b an einem Gitterelement 10, wenn sich ein Phasenobjekt 4 auf dem Gitterelement 10 befindet. Beim Eintritt in das Phasenobjekt 4 wird das ankommende Licht 20b dabei zunächst an der Oberfläche 435 des Phasenobjekts 4 gebrochen. Nach der Reflexion an dem Gitterelement 10 tritt das austretende Licht 25b wieder durch die Oberfläche 435 des Phasenobjekts 4 hindurch und wird dort erneut gebrochen. Bei einer Höhe h des Phasenobjekts 4, einem Einfallwinkel $\alpha$ des ankommenden Lichts 20b und einem Winkel $\beta$ zwischen dem Lichtstrahl und der Gitternormalen nach der Brechung an der Oberfläche 435 des Phasenobjekts 4 ergibt sich:

(1)

$$\cos(\beta) = h / s_2$$

(1a)

$$\Rightarrow \quad \underline{s_2 = h / \cos(\beta)}$$

(2)

$$\cos(\alpha - \beta) = s_1 / s_2$$

(2a)

$$\Rightarrow \quad \underline{s_1 = \cos(\alpha - \beta) \cdot s_2.}$$

Der Gangunterschied des Lichts, der sich durch die Anwesenheit des Phasenobjekts 4 auf dem Gitterelement 10 ergibt, beträgt somit:

(3)

$$\Delta g = 2 \cdot \Delta s = 2 \cdot (s_2 - s_1) \; = 2 \cdot h / \cos(\beta)(1 - \cos(\alpha - \beta))$$

Der Einfallwinkel $\alpha$ des ankommenden Lichts ist dabei durch die relative Anordnung der Lichtquelle und des Gitters vorgegeben. Wenn die Lichtquelle, beispielsweise ein Laser, fest montiert ist, und die Aufnahme für den Sensor mit dem Substrat und dem Gitter eine eindeutige Winkelorientierung der Gitterfläche relativ zu der Lichtquelle vorgibt, ist somit auch der Winkel $\alpha$ fest vorgegeben.

Der Winkel $\beta$ hängt ferner nach dem Snelliusschen Brechungsgesetz von dem Einfallwinkel $\alpha$ sowie von dem Verhältnis der Brechungsindizes des Phasenobjekts $n_2$ und des Umgebungsmediums $n_1$ ab. Dieses Verhältnis kann beispielsweise während der Messung der Motilität von biologischen Zellen konstant sein. Abweichungen können sich allerdings beispielsweise dadurch ergeben, dass dem Umgebungsmedium während der Messung ein Wirkstoff zugesetzt wird, so dass sich $n_1$ ändert.

Darüber hinaus beeinflusst die Höhe h des Phasenobjekts 4 den Gangunterschied und somit das Beugungsbild des austretenden Lichts. Eine Veränderung der Höhe h des Phasenobjekts über dem Gitterelement 10 zeigt jedoch gerade eine Bewegung des Phasenobjekts 4 an, die mit Hilfe des Beugungsbildes detektiert werden soll. Wenn beispielsweise bei einem aktuellen Beugungsbild über einem Gitterelement eine größere Höhe h vorliegt als bei einem vorangehenden Beugungsbild, kann dies bedeuten, dass sich das Phasenobjekt 4 gerade über das entsprechende Gitterelement bewegt.

Ferner haben die Brechungsindizes der Phasenobjekte und des Umgebungsmediums einen entscheidenden Einfluss auf das entstehende Beugungsbild. Eine Veränderung des Brechungsindex eines Phasenobjekts verändert beispielsweise auch ohne Veränderung der äußeren Form und ohne eine Bewegung des Phasenobjekts die optische Weglänge des Lichts und bewirkt somit eine Veränderung der Phasenlage. Darüber hinaus wird das Beugungsbild auch durch die Oberflächenkrümmung des Phasenobjekts beeinflusst. Durch eine lokale Änderung der Oberflächenkrümmung wird der Einfallswinkel des Lichts relativ zur Oberfläche verändert, so dass sich auch der Brechungswinkel an der Oberfläche und somit der Verlauf des Lichts innerhalb des Phasenobjekts verändert. Auch eine Veränderung der Oberflächenkrümmung in dem Bereich, in dem das Licht aus dem Phasenobjekt austritt, verändert das Beugungsbild.

In vielen Anwendungen kann es jedoch vorkommen, dass man gar nicht an dem konkreten Verhalten einzelner Phasenobjekte, z.B. einzelner Zellen, interessiert ist, sondern vielmehr an dem statistischen Verhalten einer Vielzahl von Phasenobjekten. Ein Beispiel hierfür ist die Untersuchung, wie sich die Zugabe eines bestimmten Wirkstoffs, beispielsweise eines Medikaments, auf die Beweglichkeit der Zellen auswirkt. In diesem Fall wäre man also nicht an der aktuellen Lage eines Phasenobjekts 4 interessiert, sondern beispielsweise an einem Durchschnittswert einer Veränderung der Bewegung mehrerer Phasenobjekte.

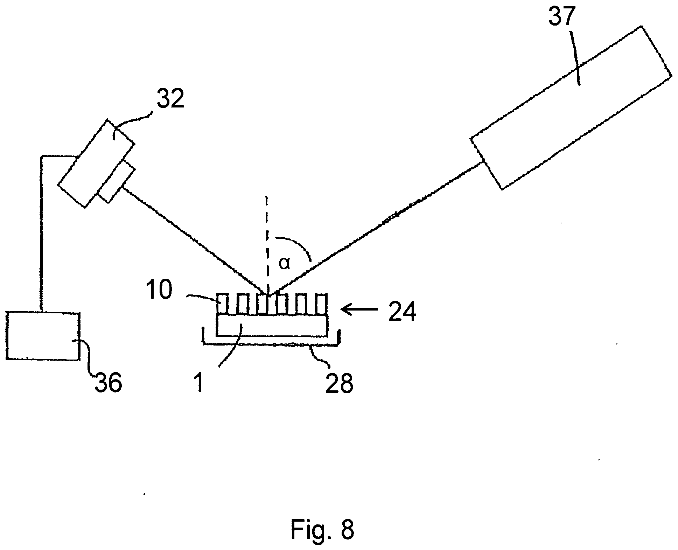

Hierzu wird erfindungsgemäß statt der herkömmlichen direkten optischen Mikroskop-Abbildung ein Beugungsbild des Gitters mit den darauf platzierten Phasenobjekten erzeugt und delektiert. Zu diesem Zweck wird eine Vorrichtung verwendet, wie sie gemäß einer Ausführungsform schematisch in Fig. 8 gezeigt ist. Die Vorrichtung umfasst eine Aufnahme 28, die zur Aufnahme des Sensors 24 geeignet ist. Die Aufnahme 28 kann irgendein Abschnitt oder irgendeine Komponente sein, auf der oder in der der Sensor 24 angeordnet werden kann. Beispielsweise kann die Aufnahme 28 ein Behälter oder eine Probenkammer sein, wie oben beschrieben worden ist. Der Sensor 24 umfasst ferner mindestens ein Substrat 1 und mindestens ein darauf angeordnetes Gitter aus Elementen 10.

Ferner umfasst die Vorrichtung eine Lichtquelle 37, bei der es sich beispielsweise um einen Laser handelt. Die Lichtquelle 37 muss eine Kohärenzlänge haben, die zumindest einen mittleren Abstand benachbarter Gitterelemente 10 übersteigt, um ein aussagekräftiges Beugungsbild zu erzeugen. Dies ist jedoch bei den mikroskopischen bzw. nanoskopischen Abmessungen des Gitters keine besonders strenge Anforderung an die Lichtquelle 37, so dass die Verwendung eines Lasers als Lichtquelle 37 nicht zwingend ist, sondern auch andere Lichtquellen in Betracht kommen. Die Lichtquelle 37 ist, wie in Fig. 8 illustriert ist, in einem Winkel $\alpha$ in Bezug auf eine durch das Gitter definierte Ebene angeordnet.

Ferner umfasst die Vorrichtung einen optischen Detektor 38, der dazu geeignet ist, zumindest einen Teil des Beugungsbildes zu empfangen und zu detektieren. Der optische Detektor 38 kann beispielsweise durch eine CCD-Kamera oder eine Photodiode gebildet sein. Der optische Detektor 38, die Aufnahme 28 und die Lichtquelle 37 können so positioniert sein, dass der optische Detektor 38 ausgewählte Hauptmaxima und/oder Nebenmaxima des Beugungsbildes detektiert und deren Intensitätsmessung gestattet. Der optische Detektor 38 wandelt das von ihm detektierte Beugungsbild oder den von ihm detektierten Teil des Beugungsbilds in ein Signal, beispielsweise ein elektrisches Signal, um. Außerdem enthält die Vorrichtung eine Auswerteschaltung 36, die an den optischen Detektor 38 gekoppelt ist, und die das Signal empfängt und aus dem Signal die Informationen bezüglich des einen oder der mehreren Phasenobjekte ermittelt. Insbesondere kann die Auswerteschaltung 36 eine Abfolge von Signalen, die von dem optischen Detektor basierend auf einer zeitlichen Abfolge aufeinanderfolgender Beugungsbilder oder aufeinanderfolgender Teile von Beugungsbildern, die durch den optischen Detektor 38 detektiert wurden, erzeugt wurden, analysieren, um daraus Informationen bezüglich des einen oder der mehreren Phasenobjekte zu ermitteln, die auf dem Gitter des Sensors 24 angeordnet sind. Beispielsweise kann eine Dichte von Phasenobjekten, z.B. eine Zelldichte ermittelt werden. Alternativ oder zusätzlich können ein Grad der Bedeckung des Gitters durch das eine oder die mehreren Phasenobjekte, eine Anzahl der Phasenobjekte, eine Veränderung des Grads der Bedeckung oder der Anzahl der Phasenobjekte und/oder eine Bewegung, eine Beweglichkeit, ein Typ, eine Form, eine Oberflächenveränderung, eine Formänderung, ein Brechungsindex, ein Durchmischungsgrad und/oder eine chemische Zusammensetzung des einen oder der mehreren Phasenobjekte ermittelt werden.

Optional kann die Vorrichtung einen Datenspeicher (nicht gezeigt) enthalten, der an die Auswerteschaltung 36 gekoppelt ist. Der Datenspeicher kann eine Datenbank speichern. Die Auswerteschaltung kann dazu eingerichtet sein, anhand des Signals eine Charakteristik des einen oder der mehreren Phasenobjekte zu ermitteln, und die ermittelte Charakteristik mit Einträgen in der Datenbank zu vergleichen, um die Informationen bezüglich des einen oder der mehreren Phasenobjekte zu ermitteln. Die auf diese Weise ermittelten Informationen können sich beispielsweise auf einen Typ einer oder mehrerer Zellen, eine Zelldichte, eine Zellaktivität, insbesondere eine Zellbewegung und/oder eine Zelloberflächenänderung, einen Brechungsindex, eine Flüssigkeitsbewegung, einen Wirkstoff, eine chemische Reaktion o.ä. beziehen.

Auch wenn in Fig. 8 schematisch eine Detektion des Beugungsbildes in Reflexion gezeigt ist, kann in alternativen Ausführungsformen das Beugungsbild oder ein Teil davon zusätzlich oder alternativ in Transmission detektiert werden, indem der optische Detektor oder ein Bereich davon entsprechend platziert wird, um durch das Substrat 1 verlaufendes Licht zu detektieren. In einigen Ausführungsformen kann alternativ oder zusätzlich eine Fourier-Optik (nicht gezeigt) im Lichtverlauf zwischen dem Sensor 24 und dem optischen Detektor 38 vorgesehen sein.

Die Entfernung zwischen dem optischen Detektor 38 und dem Gitter kann beispielsweise im ein- bis zweistelligen Zentimeterbereich liegen. Verglichen mit der Größenordnung und den Abständen der Gitterelemente 10 handelt es sich hierbei also um eine große Entfernung, so dass das Beugungsbild im Bereich des optischen Detektor 38 dem Fernfeld bzw. dem Fraunhofer-Beugungsmuster entspricht, welches seinerseits der zweidimensionalen Fouriertransformierten der Feldverteilung unmittelbar nach der Beugungsstruktur entspricht. Dies würde selbst in einer miniaturisierten Ausführungsform noch mit guter Genauigkeit gelten, wenn die Entfernung zwischen dem Gitter und dem optischen Detektor 38 lediglich im zweistelligen $\mu$m-Bereich läge.

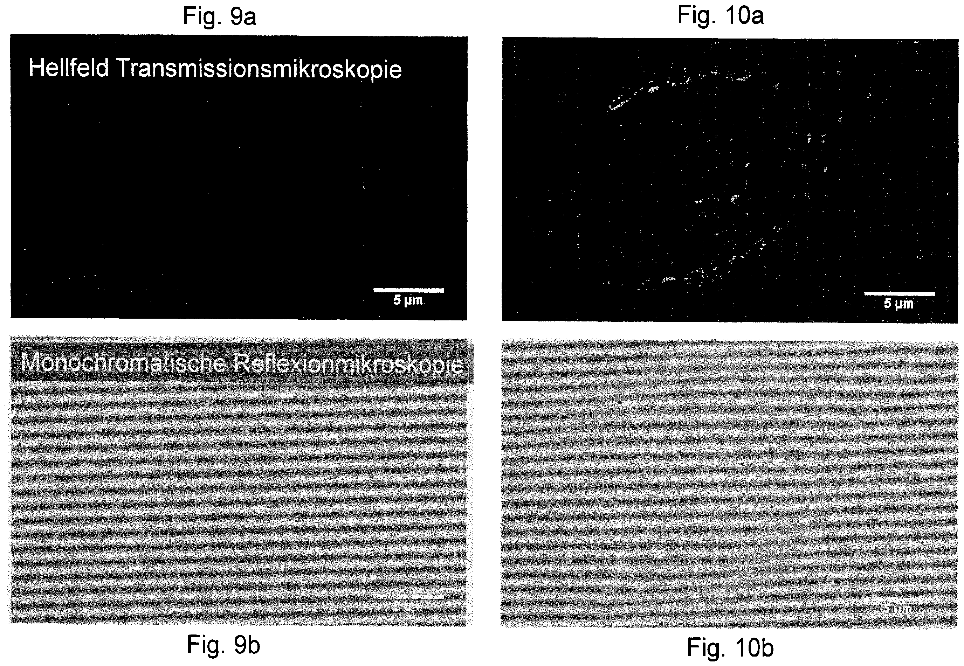

Die Fig. 9a, b und 10a, b zeigen jeweils Mikroskop-Aufnahmen, wobei die Fig. 9a, b ein Gitter ohne Phasenobjekte zeigen und Fig. 10a, b ein Gitter mit einer darauf angeordneten Zelle zeigen. Fig. 9a und 10a zeigen dabei jeweils eine Transmissions-Mikroskop-Aufnahme im Hellfeld. Während in Fig. 9a keine Strukturen erkennbar sind, zeigt Fig. 10a deutlich die Umrisse einer Zelle.

Fig. 9b und 10b zeigen jeweils eine monochromatische Mikroskop-Aufnahme in Reflexion, d.h. nach Beugung an dem Gitter. In Fig. 9b ist die regelmäßige Gitterstruktur eines (in diesem Falle) eindimensionalen Gitters ohne Störungen der Regelmäßigkeit zu erkennen. Im Vergleich hierzu zeigt Fig. 10b einige Unregelmäßigkeiten der balkenförmigen Strukturen, die gerade dem Umriss der Zelle entsprechen, wie sie auch in Fig. 10a zu sehen ist. Aufgrund der deutlichen Verformung der Balken ist die Lage der Zelle in Fig. 10b deutlich zu erkennen.

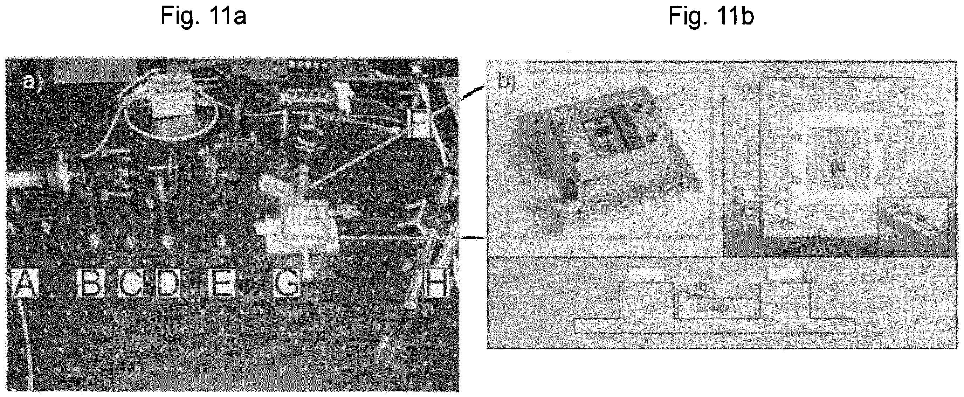

Fig. 11a zeigt eine Vorrichtung zur Untersuchung eines oder mehrerer Phasenobjekte gemäß einer Ausführungsform. Dabei ist eine Lichtquelle in Form eines Diodenlasers A vorgesehen. Im Lichtweg nach dem Diodenlaser A sind ein Shutter B, ein Polarisationsfilter C, ein Filterrad D und ein Strahlteiler E vorgesehen. Darüber hinaus umfasst die Vorrichtung zwei optische Detektoren F, H. Durch das Vorsehen von zwei optischen Detektoren F, H kann das sich einstellende Beugungsbild beispielsweise mit einem Beugungsbild nach Beugung an einem regelmäßigen Gitter ohne Phasenobjekt verglichen werden. Darüber hinaus umfasst die in Fig. 11a dargestellte Vorrichtung auch einen Probenhalter G, der beispielsweise eine Aufnahme für einen Sensor umfassen kann.

Fig. 11b zeigt den Probenhalter G der Fig. 11a im Detail. In Fig. 11b ist links oben eine dreidimensionale Ansicht, rechts oben eine Draufsicht und unten ein Querschnitt des Probenhalters G gezeigt. Wie in Fig. 11b links oben und rechts oben zu sehen ist, umfasst der Probenhalter einen Einlass und einen Auslass, die jeweils mit Schläuchen verbunden sind. Über den Einlass und den Auslass kann ein Umgebungsmedium oder ein Wirkstoff in die Aufnahme, die durch die Probenkammer gebildet ist, eingeführt werden. Innerhalb der Probenkammer befindet sich ein Sensor mit einem Gitter, das auf einem Substrat angeordnet ist. Der untere Bereich der Fig. 11b zeigt einen Querschnitt durch den Probenhalter G. Dabei ist ein Einsatz dargestellt, der das Gitter und das Substrat umfasst. Auf dem Gitter befinden sich ferner biologische Zellen, deren Reaktion auf die Wirkstoffe untersucht werden soll. Insbesondere soll im dargestellten Fall untersucht werden, wie sich die Motilität der Zellen durch die Zugabe des Wirkstoffs verändert.

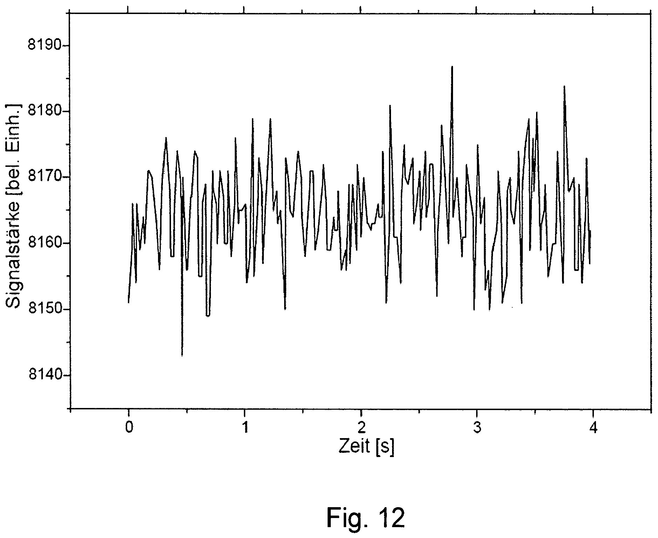

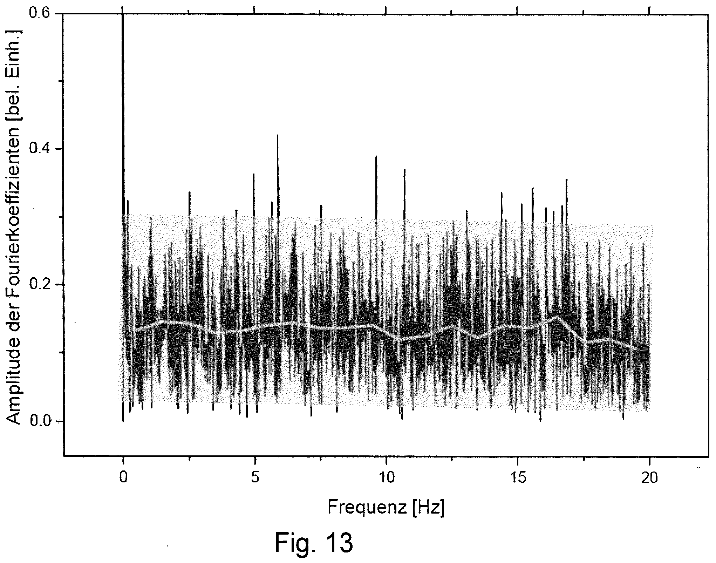

Fig. 12 zeigt den zeitlichen Verlauf der an dem optischen Detektor detektierten Signalstärke, in diesem der Intensität der Bragg-Reflexe erster Ordnung. Fig. 13 zeigt im Vergleich hierzu eine Fourier-Analyse des zeitlichen Signals, das in Fig. 12 dargestellt ist.

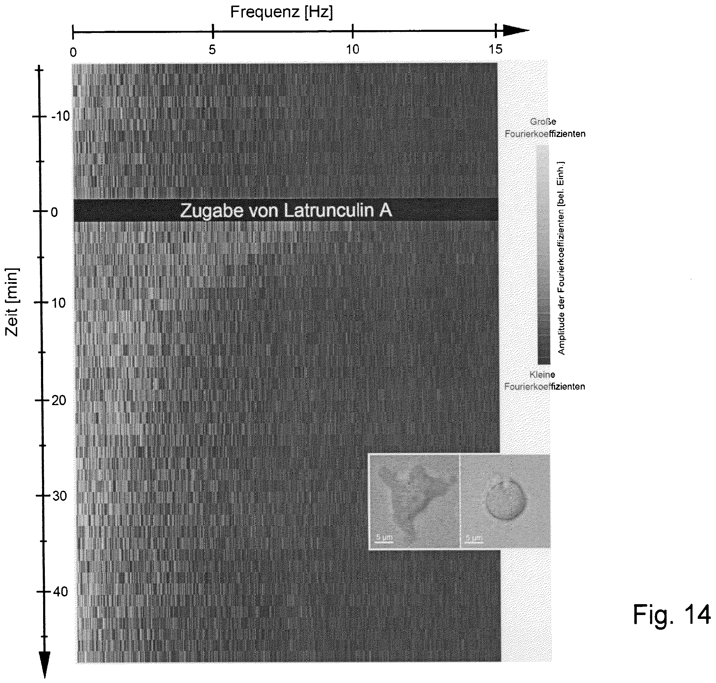

Fig. 14 zeigt einen zeitlichen Verlauf der Fourier-Koeffizienten. Dabei verläuft die Zeitachse von oben nach unten und die Frequenzachse von links nach rechts. Zum Zeitpunkt t=0 wird der Wirkstoff Latrunculin A in das in der Probenkammer befindliche Medium eingeführt. In Fig. 14 ist in Reaktion hierauf zu diesem Zeitpunkt deutlich ein Anstieg der Fourier-Koeffizienten zu erkennen, welche mit zunehmender Einwirkzeit wieder abklingen. Die eingefügte Abbildung rechts unten in Fig. 14 zeigt eine Lichtmikroskop-Aufnahme einer Dictyostelium discoideum-Zelle vor (links) und nach (rechts) der Zugabe von Latrunculin A, die auf dem Gitter des Sensors angeordnet ist.

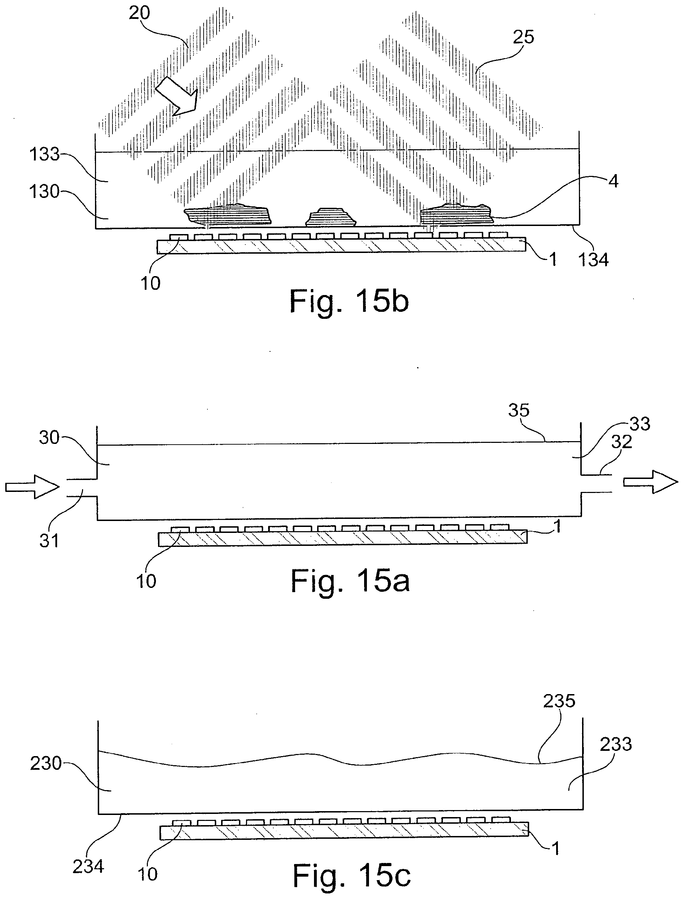

Fig. 15a-c zeigen Ausführungsformen, die den in Fig. 3-5 gezeigten Ausführungsformen ähnlich sind, bei denen sich das Gitter jedoch außerhalb des Behälters befindet. Hierdurch wird ein direkter Kontakt zwischen den Phasenobjekten und den Gitterelementen vermieden. Einerseits werden hierdurch die Gitterelemente beispielsweise vor Korrosion geschützt. Andererseits wird aber auch vermieden, dass die Phasenobjekte, bei denen es sich beispielsweise um biologische Zellen handelt, durch das Material der Gitterelemente beeinflusst werden. Da die Anordnungen in den Fig. 15a-c im Übrigen denjenigen der Fig. 3-5 entsprechen, wird auf die obige detaillierte Beschreibung verwiesen, um Wiederholungen zu vermeiden. Um einen Vergleich zu erleichtern, sind in den Fig. 15a-c ferner die gleichen Bezugszeichen verwendet worden wie in den Fig. 3-5. Während das Gitter in den Ausführungsformen der Fig. 15a-c jeweils unter dem Boden des Behälters angeordnet ist, ist anzumerken, dass in anderen Ausführungsformen das Gitter auch seitlich neben dem Behälter oder darüber angeordnet sein kann, je nach Anordnung der Lichtquelle und Ausrichtung des eingestrahlten Beleuchtungslichts.

Die in Fig. 15a-c gezeigte Ausführungsform, bei der die Phasenobjekte in einem separaten, von dem Gitter getrennten Behälter angeordnet sind, hat eine Reihe praktischer Vorteile. Ein Vorteil besteht darin, dass dieser Aufbau einen variablen Abstand zwischen dem Probengefäß und dem Gitter erlaubt. Wenn der in der Darstellung von Fig. 15 vertikale Abstand zwischen dem Probengefäß und dem Gitter groß genug ist, kann erreicht werden, dass das Licht die Probe nur einmal durchläuft, bevor es am Detektor detektiert wird. Auch ist es möglich, die Lichtquelle, das Gitter und den Detektor in einer justierten Position festzuhalten, und dann nacheinander unterschiedliche Probenbehälter in den Lichtweg zu bringen, ohne dass erneute Justagen erforderlich werden. Der Austausch der Probenbehälter kann teilweise oder vollständig automatisch erfolgen, wodurch der Durchsatz erhöht wird.

Modifikationen der beschriebenen Ausführungsformen sind möglich. Beispielsweise kann alternativ oder zusätzlich zu dem Beugungsbild, das durch an den Gitterelementen reflektierte Lichtstrahlen gebildet wird, das Beugungsbild, das durch zwischen den Gitterelementen hindurchtretende Lichtstrahlen gebildet wird, detektiert werden. In einigen Ausführungsformen sind mehrere optische Detektoren vorgesehen, um unterschiedliche Teile des Beugungsbildes zu detektieren.

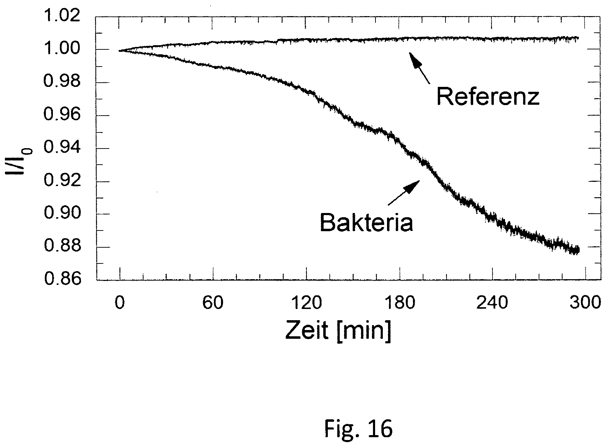

Fig. 16 zeigt die normierte Intensität eines Beugungsmaximums als Funktion der Zeit. Eine Referenzkurve zeigt die normierte Intensität in einem Maximum des Beugungsbildes des an dem Gitter gestreuten Beleuchtungslichtes ohne Bakterien. Eine weitere Kurve zeigt die normierte Intensität in einem Fall, bei dem Bakterien in einer Nährlösung als Phasenobjekte im Lichtweg angeordnet sind. Man erkennt in Fig. 16, dass die Intensität des Beugungsmaximums bei der Bakterienprobe mit der Zeit abnimmt. Diese Abnahme ist auf eine fortschreitende Teilung der Bakterien, d. h. ein Anwachsen der Bakterienpopulation zurückzuführen, d. h. auf eine Zunahme der Anzahl von Phasenobjekten, die die konstruktive Interferenz im Beugungsmaximum zunehmend stören und daher zu einem Absinken der Intensität führen. Ferner erkennt man in Fig. 16 dass die Intensität mit zunehmender Zeit in zunehmendem Maße fluktuiert, was auf eine Aktivität der Zellen zurückgeführt werden kann.

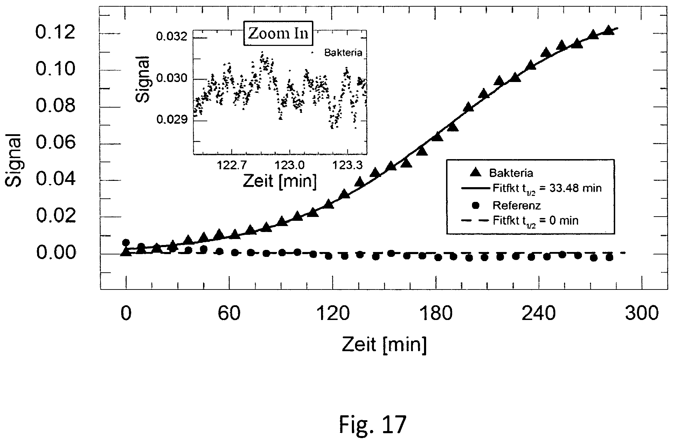

Fig. 17 zeigt Messpunkte eines Signals, welches der Differenz zwischen der Referenzintensität und der Intensität mit Bakterien entspricht. Es sind lediglich einzelne Messpunkte gezeigt, um gleichzeitig eine durchgezogene Fitfunktionskurve darstellen zu können, die eine bestimmte Teilungsrate, im vorliegenden Fall 33,48min repräsentiert. Ferner ist in Fig. 17 ein beispielhafter vergrößerter Ausschnitt des Verlaufs der Messsignale gezeigt, in dem man die Fluktuation des Signals gut erkennen kann. Durch Fitten einer Wachstumskurve, die als Parameter die Teilungsrate enthält, kann auf diese Weise die Teilungsrate der Bakterien aus dem Signal abgeleitet werden.

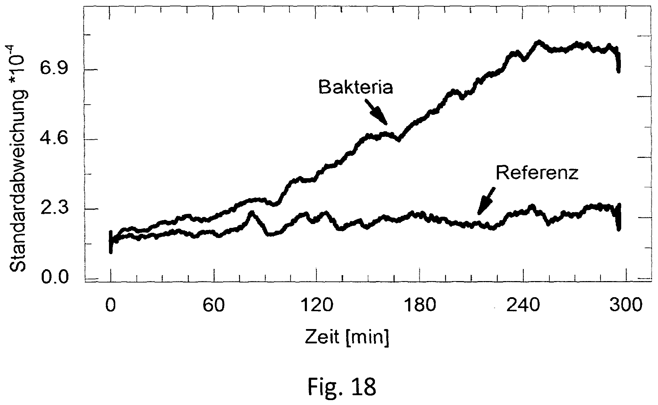

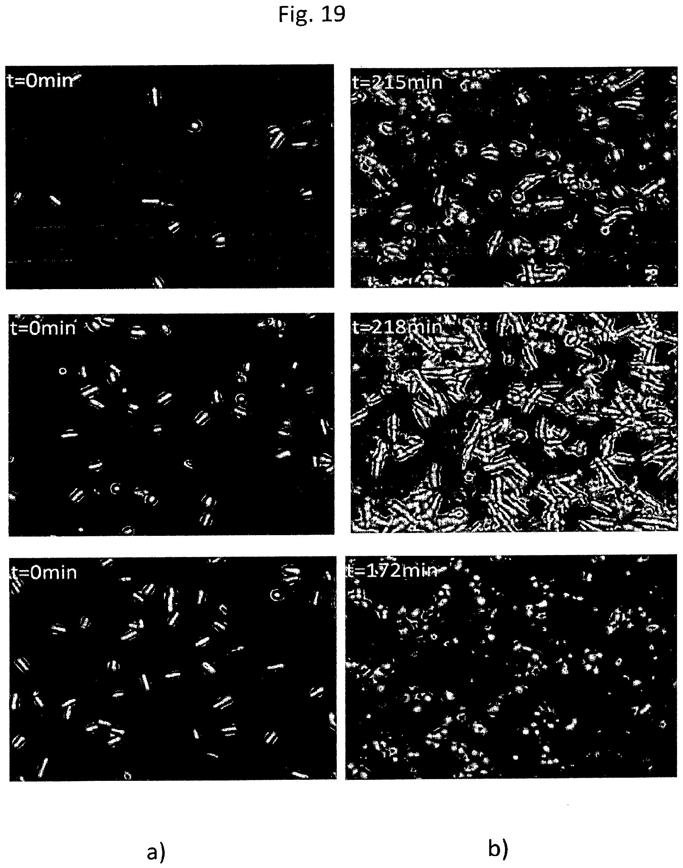

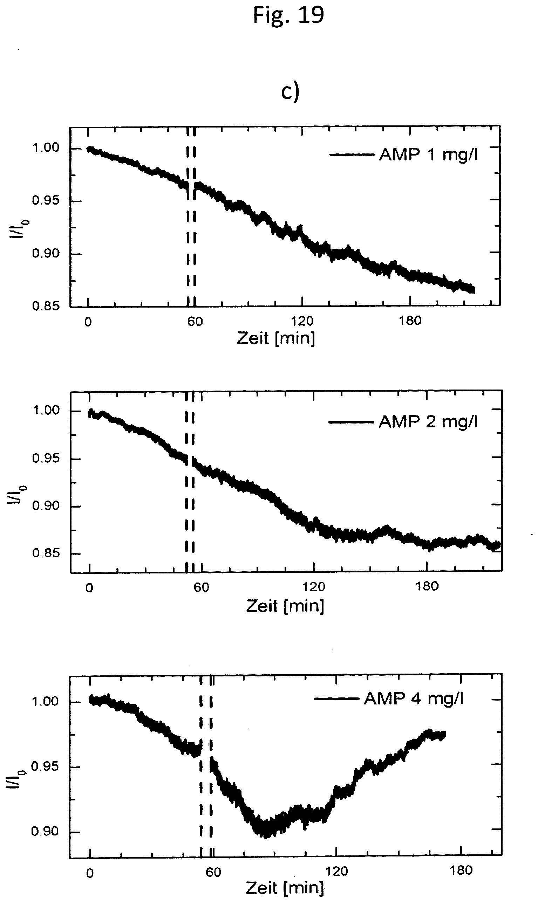

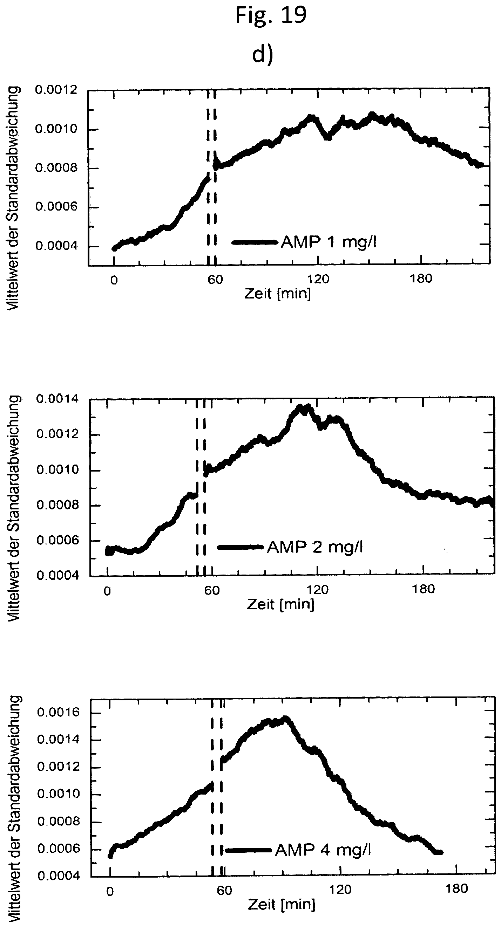

Fig. 18 zeigt den zeitlichen Verlauf der Fluktuation in der Intensität, die hier durch die Standardabweichung der Signale von einer glatten Fitkurve repräsentiert sind. Wie Fig. 18 zu entnehmen ist, nimmt die Standardabweichung kontinuierlich zu. Die Standardabweichung repräsentiert Fluktuationen des Signals auf einer kurzen Zeitskala, die ihrerseits als ein Maß für die Aktivität der Bakterien betrachtet werden können, was unter Bezugnahme auf Fig. 19 näher erläutert wird.

Fig. 19 zeigt in Spalte a) optische Mikroskopbilder einer Probe zum Zeitpunkt 0 mit einer bestimmten Ausgangs-Bakterienpopulation. Die Spalte b) zeigt optische Mikroskopbilder zu späteren Zeitpunkten, nämlich nach 215min in der ersten Zeile, nach 208min in der zweiten Zeile und nach 172min in der dritten Zeile. Die Spalte c) zeigt die normierte Intensität als Funktion der Zeit und die Spalte d) die Mittelwerte der Standardabweichung als Funktion der Zeit.

Die erste Zeile von Fig. 19 zeigt einen Fall, bei dem die Bakterien nach ca. 60min Wachstum mit einem Antibiotikum AMP mit einer Konzentration von 1mg/l behandelt wurden. Man erkennt, dass auch nach der Behandlung mit dem Antibiotikum die normierte Intensität abfällt, was für ein weiteres Wachstum der Bakterienpopulation spricht. Gleichzeitig ist dabei in Zeile 1, Spalte d) erkennbar, dass abweichend von der Situation von Fig. 18, die Standardabweichung etwa bei 120min ein Plateau erreicht und danach wieder abfällt, was für ein Absterben der Zellen spricht.

Die zweite Spalte zeigt eine ähnliche Situation, nur dass eine doppelte Menge von 2mg/l AMP zugegeben wird. Man erkennt, dass die Abnahme der normierten Intensität etwa bei 120min abflacht, d. h. dass in diesem Zeitbereich das Wachstum der Zellen stark verlangsamt wird. Gleichzeitig ist in Zeile 2, Spalte d) zu erkennen, dass etwa bei 120min die Standardabweichung sehr deutlich abfällt, was kennzeichnend für das Absterben der Zellen ist.

Die dritte Zeile zeigt einen ähnlichen Verlauf, nur dass bei etwa 60min eine noch größere Dosis an Antibiotikum AMP von 4mg/l zugegeben wird. In der dritten Zeile, Spalte d) erkennt man wiederum ein rapides Abfallen der Standardabweichung, das noch ausgeprägter ist als in der zweiten Zeile, was in Anbetracht der doppelten Dosis an Antibiotikum der Erwartung entspricht. Zusätzlich erkennt man in Zeile 3, Spalte c), dass die Intensität nach etwa 90min wieder beginnt anzusteigen. Dies ist ein Zeichen dafür, dass die Zellen aufgrund der hohen Antibiotikakonzentration nicht nur absterben, sondern sich auch zumindest teilweise auflösen, was durch den Vergleich der Mikroskopbilder in Spalte b) auch gut zu erkennen ist. Die sich auflösenden Bakterien verlieren ihre Funktion als "Phasenobjekte", wodurch die Intensität im Beugungsmaximum wieder zunimmt.

BEZUGSZEICHENLISTE

| 1 | Substrat |
|---|---|
| 10, 10', 10a-d | Gitterelement |
| 20, 20a-d | ankommendes Licht |
| 25, 25a-d | austretendes Licht |
| 24 | Sensor |
| 28 | Aufnahme |
| 130 | Behälter |
| 133 | Fluid |
| 134 | Boden |

| | | |
|---|---|---|
| 230 | Behälter |
| 233 | Fluid |
| 234 | Boden |
| 235 | Oberfläche |
| 30 | Behälter |
| 31 | Einlass |
| 32 | Auslass |
| 33 | Fluid |
| 34 | Boden |
| 35 | Oberfläche |
| 36 | Auswerteschaltung |
| 37 | Lichtquelle |
| 38 | optischer Detektor |
| 4 | Phasenobjekt |
| 435 | Oberfläche |
| A | Laser |
| B | Shutter |
| C | Polarisationsfilter |
| D | Filterrad |
| E | Strahlteiler |
| F, H | optische Detektoren |
| G | Probenhalter |
| $\alpha$, $\beta$ | Winkel |
| $s_1$, $s_2$ | Strecken |
| h | Höhe |

**Patentansprüche**

1. Verfahren zur Untersuchung eines oder mehrerer Phasenobjekte (4; 33; 233), wobei ein Gitter aus Elementen (10; 10'; 10a-d) verwendet wird, das mit Licht (20; 20a-d) einer Lichtquelle (37) beleuchtet wird, dessen Kohärenzlänge größer als der mittlere Abstand benachbarter Elemente (10; 10'; 10a-d) des Gitters ist, wobei ein Beugungsbild des an dem Gitter gestreuten Beleuchtungslichtes erzeugt wird, wobei das eine oder die mehreren Phasenobjekte (4; 33; 233) im Lichtweg zwischen der Lichtquelle (37) und dem Gitter und/oder im Lichtweg des an dem Gitter gestreuten Beleuchtungslichts platziert sind, wobei zumindest ein Teil des Beugungsbildes direkt oder nach Wechselwirkung mit weiteren optischen Komponenten von einem optischen Detektor (38) detektiert wird, wobei der optische Detektor (38) das detektierte Beugungsbild oder den detektierten Teil des Beugungsbildes in ein Signal umwandelt, wobei das Signal weiter analysiert wird, um daraus Informationen bezüglich des einen oder der mehreren Phasenobjekte (4; 33; 233) zu ermitteln.

2. Verfahren nach Anspruch 1, bei dem der optische Detektor (38) eine zeitliche Abfolge von Beugungsbildern oder Teilen von Beugungsbildern detektiert und in eine Abfolge von Signalen umwandelt, wobei die Abfolge der Signale weiter analysiert wird, um daraus die Informationen bezüglich des einen oder der mehreren Phasenobjekte (4; 33; 233) zu ermitteln, und/oder bei dem zumindest zwei Teile des Beugungsbildes von zumindest zwei räumlich voneinander getrennten optischen Detektoren gleichzeitig detektiert und in Signale umgewandelt werden, wobei die Signale weiter analysiert werden, um daraus die Informationen bezüglich des einen oder der mehreren Phasenobjekte zu ermitteln.

3. Verfahren nach Anspruch 1 oder 2, bei dem zumindest die Intensität eines Hauptmaximums des Beugungsbildes von dem optischen Detektor (38) detektiert wird, und/oder bei dem zwischen dem Gitter und dem einen oder den mehreren Phasenobjekten eine transparente Schutzschicht angeordnet ist, und/oder bei dem das Gitter und die Phasenobjekte voneinander beabstandet sind, wobei die Phasenobjekte vorzugsweise in einem von dem Gitter getrennten Behälter untergebracht sind.

4. Verfahren nach einem der vorhergehenden Ansprüche, bei dem die Phasenobjekte (4) biologische Zellen oder Kleinsttiere sind, und/oder bei dem aufgrund des Signals eine Veränderung des einen oder der mehreren Phasenobjekte (4; 33; 233) ermittelt wird, und/oder bei dem das eine oder die mehreren Phasenobjekte (4) ferner einer Substanz und/oder einem physikalischen Reiz ausgesetzt werden, und/oder bei dem aufgrund des Signals eine Oberflächen- und/oder Volumenänderung des einen oder der mehreren Phasenobjekte (233), ein Prozess innerhalb des einen oder der mehreren Phasenobjekte, eine Änderung der Dichte des einen oder der mehreren Phasenobjekte und/oder eine Bewegung des einen oder der mehreren Phasenobjekte relativ zu dem Gitter ermittelt wird, und/oder bei dem aufgrund des Signals ein Grad einer Durchmischung von zumindest zwei Phasenobjekten (33) ermittelt wird, und/oder bei dem aufgrund des Signals ein Grad einer Bedeckung des Gitters durch das eine oder die mehreren Phasenobjekte (4), eine Anzahl der Phasenobjekte (4) und/oder eine Veränderung des Grads der Bedeckung oder der Anzahl der Phasenobjekte (4) ermittelt wird.

5. Verfahren nach einem der vorhergehenden Ansprüche, bei dem das Beugungsbild oder der Teil des Beugungsbildes zumindest teilweise in Transmission durch das Gitter detektiert wird, oder bei dem das Beugungsbild oder der Teil des Beugungsbildes zumindest teilweise in Reflektion an dem Gitter detektiert wird.

6. Verfahren nach einem der vorhergehenden Ansprüche, bei dem die Elemente des Gitters periodisch oder quasiperiodisch angeordnet sind, und/oder bei dem das Gitter ein zweidimensionales Gitter ist.

7. Verfahren nach Anspruch 2 oder einem davon abhängigen Anspruch, bei dem die zeitliche Abfolge zumindest drei, insbesondere zumindest 20 und bevorzugt zumindest 100 zeitlich nacheinander detektierte Beugungsbilder oder detektierte Teile von Beugungsbildern umfasst, und/oder bei dem durch eine Verarbeitung der Abfolge der Signale eine Korrelation, insbesondere eine Fourieranalyse der zeitlichen Abfolge der detektierten Beugungsbilder oder der detektierten Teile von Beugungsbildern durchgeführt wird, und/oder bei dem der zeitliche Verlauf einer Intensität eines Abschnittes der Beugungsbilder und/oder die zeitliche Fluktuation der Intensität eines Abschnittes der Beugungsbilder ermittelt wird.

8. Verfahren nach einem der vorhergehenden Ansprüche, bei dem anhand des Signals eine Charakteristik des einen oder der mehreren Phasenobjekte (4; 33; 233) ermittelt wird, und die ermittelte Charakteristik mit Einträgen in einer Datenbank verglichen wird, um die Informationen bezüglich des einen oder der mehreren Phasenobjekte, insbesondere einen Grad der Bedeckung des Gitters durch das eine oder die mehreren Phasenobjekte, eine Anzahl der Phasenobjekte (4), eine Veränderung des Grads der Bedeckung oder der Anzahl der Phasenobjekte (4) und/oder eine Bewegung, eine Beweglichkeit, einen Typ, eine Form, eine Oberflächenveränderung, eine Formänderung, einen Brechungsindex, eine Dichteänderung, eine Volumenänderung, eine auf das eine oder die mehreren Phasenobjekte ausgeübte Kraft, eine von dem einen oder den mehreren Phasenobjekten ausgeübte Kraft, eine Substanz, der das eine oder die mehreren Phasenobjekte ausgesetzt sind, einen physikalischen Reiz, dem das eine oder die mehreren Phasenobjekte ausgesetzt sind, einen Durchmischungsgrad und/oder eine chemische Zusammensetzung des einen oder der mehreren Phasenobjekte zu ermitteln, und/oder bei dem auf oder unter dem Gitter eine dreidimensionale Matrix angeordnet ist, auf, unter oder in welcher das eine oder die mehreren Phasenobjekte angeordnet sind.

9. Vorrichtung zur Untersuchung eines oder mehrerer Phasenobjekte (4; 33; 233), umfassend:

eine Aufnahme (28; 30; 130, 230) zum Aufnehmen eines Sensors (24) mit einem Gitter aus Elementen (10; 10'; 10a-d),
eine Lichtquelle (37), die so angeordnet oder anordenbar ist, dass sie einen in oder auf der Aufnahme (28; 30; 130, 230) angeordneten Sensor (24) derart beleuchten kann, dass durch an dem Gitter gestreutes Licht (25; 25a-d) der Lichtquelle (37) ein Beugungsbild erzeugt wird und ein oder mehrere zu untersuchende Phasenobjekte (4; 33; 233) im Lichtweg zwischen der Lichtquelle (37) und dem Gitter und/oder im Lichtweg des an dem Gitter gestreuten Beleuchtungslichts angeordnet sind,
einen optischen Detektor (38), der dazu eingerichtet ist, zumindest einen Teil des Beugungsbildes direkt oder nach Wechselwirkung mit weiteren optischen Komponenten zu detektieren und in ein Signal umzuwandeln, und
eine Auswerteschaltung (36), die an den optischen Detektor (38) gekoppelt ist und das Signal empfängt, wobei die Auswerteschaltung (36) dazu ausgelegt ist, aus dem Signal Informationen bezüglich des einen oder der mehreren Phasenobjekte (4; 33; 233) zu ermitteln.

10. Vorrichtung nach Anspruch 9, bei welcher der optische Detektor (38) einen Bildsensor und/oder einen Photodetektor umfasst, und/oder bei welcher der optische Detektor (38) angeordnet ist, um zumindest die Intensität eines Hauptmaximums des Beugungsbildes zu detektieren.

11. Vorrichtung nach Anspruch 9 oder 10, bei welcher der optische Detektor (38) dazu eingerichtet ist, eine zeitliche Abfolge von Beugungsbildern oder Teilen von Beugungsbildern zu detektieren und in eine Abfolge von Signalen umzuwandeln, wobei die Auswerteschaltung (36) dazu ausgelegt ist, aus der Abfolge der Signale die Informationen bezüglich des einen oder der mehreren Phasenobjekte (4; 33; 233) zu ermitteln, wobei die zeitliche Abfolge vorzugsweise zumindest drei, insbesondere zumindest 20 und bevorzugt zumindest 100 zeitlich nacheinander detektierte Beugungsbilder oder detektierte Teile von Beugungsbildern umfasst, und/oder wobei die Auswerteschaltung (36) vorzugsweise dazu ausgelegt ist, durch eine Verarbeitung der Abfolge der Signale eine Korrelation, insbesondere eine Fourieranalyse der Abfolge der detektierten Beugungsbilder oder der detektierten Teile der Beugungsbilder durchzuführen.

12. Vorrichtung nach einem der Ansprüche 9 bis 11, wobei der optische Detektor ein erster optischer Detektor ist, der dazu eingerichtet ist, einen ersten Teil des Beugungsbildes zu detektieren und in ein erstes Signal umzuwandeln, und wobei die Vorrichtung einen zweiten optischen Detektor umfasst, der von dem ersten optischen Detektor räumlich getrennt ist und dazu eingerichtet ist, einen zweiten Teil des Beugungsbildes zu detektieren und in ein zweites Signal umzuwandeln, wobei die Auswerteschaltung ferner an den zweiten optischen Detektor gekoppelt ist und das zweite Signal empfängt und dazu ausgelegt ist, aus dem ersten und dem zweiten Signal die Informationen bezüglich des einen oder der mehreren Phasenobjekte zu ermitteln, und/oder bei welcher der optische Detektor (38) angeordnet ist, um das Beugungsbild oder den Teil des Beugungsbildes zumindest teilweise in Transmission durch das Gitter und/oder zumindest teilweise in Reflektion an dem Gitter zu detektieren.

13. Vorrichtung nach einem der Ansprüche 9 bis 12, bei der die Auswerteschaltung (36) dazu ausgelegt ist, aufgrund des Signals eine Veränderung des einen oder der mehreren Phasenobjekte (4; 33; 233) zu ermitteln, wobei die Auswerteschaltung (36) vorzugsweise dazu ausgelegt ist, aufgrund des Signals eine Oberflächen- und/oder Volumenänderung des einen oder der mehreren Phasenobjekte (233), einen Prozess innerhalb des einen oder der mehreren Phasenobjekte, eine Änderung der Dichte des einen oder der mehreren Phasenobjekte und/oder eine Bewegung des einen oder der mehreren Phasenobjekte relativ zu dem Gitter zu ermitteln, und/oder bei der die Auswerteschaltung (36) vorzugsweise dazu ausgelegt ist, aufgrund des Signals einen Grad einer Durchmischung von zumindest zwei Phasenobjekten (33) zu ermitteln, und/oder bei der die Auswerteschaltung (36) dazu ausgelegt ist, aufgrund des Signals einen Grad einer Bedeckung des Gitters durch das eine oder die mehreren Phasenobjekte (4), eine Anzahl der Phasenobjekte (4) und/oder eine Veränderung des Grads der Bedeckung oder der Anzahl der Phasenobjekte (4) zu ermitteln.

14. Vorrichtung nach einem der Ansprüche 9 bis 13, die ferner einen Datenspeicher mit einer darin gespeicherten Datenbank umfasst oder damit koppelbar ist, und bei der die Auswerteschaltung (36) dazu ausgelegt ist, anhand des Signals eine Charakteristik des einen oder der mehreren Phasenobjekte (4; 33; 233) zu ermitteln, und die ermittelte Charakteristik mit Einträgen in der Datenbank zu vergleichen, um die Informationen bezüglich des einen oder der mehreren Phasenobjekte, insbesondere einen Grad der Bedeckung des Gitters durch das eine oder die mehreren Phasenobjekte, eine Anzahl der Phasenobjekte (4), eine Veränderung des Grads der Bedeckung oder der Anzahl der Phasenobjekte (4) und/oder eine Bewegung, eine Beweglichkeit, einen Typ, eine Form, eine Oberflächenveränderung, eine Formänderung, einen Brechungsindex, eine Dichteänderung, eine Volumenänderung, eine auf das eine oder die mehreren Phasenobjekte ausgeübte Kraft, eine von dem einen oder den mehreren Phasenobjekten ausgeübte Kraft, eine Substanz, der das eine oder die mehreren Phasenobjekte ausgesetzt sind, einen physikalischen Reiz, dem das eine oder die mehreren Phasenobjekte ausgesetzt sind, einen Durchmischungsgrad und/oder eine chemische Zusammensetzung des einen oder der mehreren Phasenobjekte zu ermitteln, und/oder bei welcher die genannte optische Komponente eine Fourier-Optik umfasst, die angeordnet ist, um das Beugungsbild oder den Teil des Beugungsbildes in ein reelles Bild einer Anordnung des einen oder der mehreren Phasenobjekte (4; 33; 233) umzuwandeln.

15. Vorrichtung nach einem der Ansprüche 9 bis 14, die ferner einen in oder auf die Aufnahme (28; 30; 130, 230) aufgenommenen Sensor mit einem Gitter aus Elementen (10; 10'; 10a-d) enthält, bei der auf dem Gitter des Sensors vorzugsweise eine Schutzschicht angeordnet ist, um darauf das eine oder die mehreren Phasenobjekte anzuordnen, oder die vorzugsweise ferner einen Behälter zur Aufnahme des einen oder der mehreren Phasenobjekte umfasst, wobei der Behälter im Lichtweg zwischen der Lichtquelle (37) und dem Gitter und/oder im Lichtweg des an dem Gitter gestreuten Beleuchtungslichts angeordnet oder anordenbar ist, wobei sich der in oder auf der Aufnahme aufgenommene Sensor mit dem Gitter außerhalb des Behälters befindet.

**Claims**

1. A method for investigating one or a plurality of phase objects (4; 33; 233), wherein a grid made up of elements (10;

10'; 10a-d) is used, which is illuminated with light (20; 20a-d) of a light source (37), the coherence length of which is larger than the average spacing of adjacent elements (10; 10'; 10a-d) of the grid, wherein a diffraction image of the illuminating light scattered on the grid is generated, wherein the one or the plurality of phase objects (4; 33; 233) are placed in the light path between the light source (37) and the grid and/or in the light path of the illuminating light scattered on the grid,

wherein at least a part of the diffraction image is detected by an optical sensor (38) directly or after interaction with further optical components, wherein the optical detector (38) converts the detected diffraction image or the detected part of the diffraction image into a signal, wherein the signal is analysed further in order to ascertain information relating to the one or plurality of phase objects (4; 33; 233) therefrom.

2. The method according to Claim 1, in which the optical detector (38) detects a temporal sequence of diffraction images or parts of diffraction images and converts the same into a sequence of signals, wherein the sequence of signals is analysed further, in order to ascertain the information relating to the one or the plurality of phase objects (4; 33; 233) therefrom, and/or

in which at least two parts of the diffraction image are detected simultaneously by at least two mutually spatially separated optical detectors and converted into signals,

wherein the signals are analysed further, in order to ascertain the information relating to the one or the plurality of phase objects therefrom.

3. The method according to Claim 1 or 2, in which at least the intensity of a primary maximum of the diffraction image is detected by the optical detector (38), and/or

in which a transparent protective layer is arranged between the grid and the one or the plurality of phase objects, and/or

wherein the phase objects are preferably accommodated in a container which is separated from the grid.

4. The method according to one of the preceding claims, in which the phase objects (4) are biological cells or minute animals, and/or

in which a change of the one or the plurality of the phase objects (4; 33; 233) is ascertained on the basis of the signal, and/or

in which the one or the plurality of phase objects (4) are furthermore exposed to a substance and/or a physical stimulus, and/or

in which a surface and/or volume change of the one or the plurality of phase objects (233), a process within the one or the plurality of phase objects, a change of the density of the one or the plurality of phase objects and/or a movement of the one or the plurality of phase objects relatively to the grid is ascertained on the basis of the signal, and/or

in which a level of intermixing of at least two phase objects (33) is ascertained on the basis of the signal, and/or

in which a level of coverage of the grid by the one or the plurality of phase objects (4), a number of phase objects (4) and/or a change of the level of the coverage or of the number of phase objects (4) is ascertained on the basis of the signal.

5. The method according to one of the preceding claims, in which the diffraction image or the part of the diffraction image is detected at least to some extent in transmission through the grid, or

in which the diffraction image or the part of the diffraction image is detected at least to some extent in reflection on the grid.

6. The method according to one of the preceding claims, in which the elements of the grid are arranged periodically or quasi-periodically, and/or

in which the grid is a two-dimensional grid.

7. The method according to Claim 2 or a claim dependent thereon, in which the temporal sequence comprises at least three, in particular at least 20 and preferably at least 100 temporally successively detected diffraction images or detected parts of diffraction images, and/or

in which a correlation, particularly a Fourier analysis of the temporal sequence of the detected diffraction images or the detected parts of diffraction images is carried out by means of processing of the sequence of the signals, and/or

wherein the temporal evolution of an intensity of a part of the diffraction image and/or the temporal fluctuation of the intensity of a part of said diffraction image is determined.

8. The method according to one of the preceding claims, in which a characteristic of the one or the plurality of phase objects (4; 33; 233) is ascertained on the basis of the signal, and the ascertained characteristic is compared with entries in a database, in order to ascertain the information relating to the one or the plurality of phase objects, particularly a level of coverage of the grid by the one or plurality of phase objects, a number of phase objects (4), a change of the level of coverage or the number of phase objects (4) and/or a movement, a movability, a type, a shape, a surface change, a shape change, a refractive index, a density change, a volume change, a force exerted on the one or the plurality of phase objects, a force exerted by the one or the plurality of phase objects, a substance, to which the one or the plurality of phase objects are exposed, a physical stimulus, to which the one or the plurality of phase objects are exposed, a level of intermixing and/or a chemical composition of the one or the plurality of phase objects, and/or

in which a three-dimensional matrix is arranged on or below the grid, on, below or in which the one or the plurality of phase objects arc arranged.

9. A device for investigating one or a plurality of phase objects (4; 33; 233), comprising:

a receptacle (28; 30; 130, 230) for accommodating a sensor (24) with a grid made up of elements (10; 10'; 10a-d), a light source (37), which is or can be arranged in such a manner that it can illuminate a sensor (24) arranged in or on the receptacle (28; 30; 130, 230) in such a manner that a diffraction image is created by the light (25; 25a-d) of the light source (37) scattered on the grid and one or a plurality of phase objects (4; 33; 233) to be investigated are arranged in the light path between the light source (37) and the grid and/or in the light path of the illuminating light scattered at the grid,

an optical detector (38), which is configured to detect at least a part of the diffraction image directly or after interaction with further optical components and convert the same into a signal, and

an evaluation circuit (36), which is coupled to the optical detector (38) and receives the signal, wherein the evaluation circuit (36) is designed to ascertain information relating to the one or the plurality of phase objects (4; 33; 233) from the signal.

10. The device according to Claim 9, in which the optical detector (38) comprises an image sensor and/or a photodetector, and/or

in which the optical detector (38) is arranged in order to detect at least the intensity of a principal maximum of the diffraction image.

11. The device according to Claim 9 or 10, in which the optical detector (38) is configured to detect a temporal sequence of diffraction images or parts of diffraction images and to convert the same into a sequence of signals, wherein the evaluation circuit (36) is designed to ascertain the information relating to the one or the plurality of phase objects (4; 33; 233) from the sequence of the signals,

in which the temporal sequence preferably comprises at least three, in particular at least 20 and preferably at least 100 temporally successively detected diffraction images or detected parts of diffraction images, and/or

in which the evaluation circuit (36) is preferably configured to carry out a correlation, particularly a Fourier analysis of the sequence of the detected diffraction images or the detected parts of the diffraction images by means of processing of the sequence of the signals.

12. The device according to one of Claims 9 to 11, wherein the optical detector is a first optical detector, which is configured to detect a first part of the diffraction image and convert the same into a first signal, and wherein the device comprises a second optical detector, which is spatially separated from the first optical detector and is configured to detect a second part of the diffraction image and convert the same into a second signal, wherein the evaluation circuit is further coupled to the second optical detector and receives the second signal and is designed to ascertain the information relating to the one or the plurality of phase objects from the first and the second signal, and/or

in which the optical detector (38) is arranged in order to detect the diffraction image or the part of the diffraction image at least to some extent in transmission through the grid and/or at least to some extent in reflection at the grid.

13. The device according to one of Claims 9 to 12, in which the evaluation circuit (36) is configured to ascertain a change of the one or the plurality of the phase objects (4; 33; 233) on the basis of the signal,

in which the evaluation circuit (36) is preferably configured to ascertain a surface and/or volume change of the one or the plurality of phase objects (233), a process within the one or the plurality of phase objects, a change of the density of the one or the plurality of phase objects and/or a movement of the one or the plurality of phase objects relatively to the grid on the basis of the signal, and/or

in which the evaluation circuit (36) is preferably configured to ascertain a level of intermixing of at least two phase objects (33) on the basis of the signal, and/or

in which the evaluation circuit (36) is configured to ascertain a level of coverage of the grid by the one or the plurality of phase objects (4), a number of phase objects (4) and/or a change of the level of the coverage or of the number of phase objects (4) on the basis of the signal.

14. The device according to one of Claims 9 to 13, which further comprises a data memory with a database stored therein or which can be coupled to the same, and in which the evaluation circuit (36) is configured to ascertain a characteristic of the one or the plurality of phase objects (4; 33; 233) on the basis of the signal, and to compare the ascertained characteristic with entries in a database, in order to ascertain the information relating to the one or the plurality of phase objects, particularly a level of coverage of the grid by the one or plurality of phase objects, a number of phase objects (4), a change of the level of coverage or of the number of phase objects (4) and/or a movement, a movability, a type, a shape, a surface change, a shape change, a refractive index, a density change, a volume change, a force exerted on the one or the plurality of phase objects, a force exerted by the one or the plurality of phase objects, a substance, to which the one or the plurality of phase objects are exposed, a physical stimulus, to which the one or the plurality of phase objects are exposed, a level of intermixing and/or a chemical composition of the one or the plurality of phase objects, and/or
in which the optical component mentioned comprises a Fourier optical system, which is arranged in order to convert the diffraction image or the part of the diffraction image into a real image of an arrangement of the one or the plurality of phase objects (4; 33; 233).

15. The device according to one of Claims 9 to 14, which further contains a sensor with a grid made up of elements (10; 10'; 10a-d), which is accommodated in or on the receptacle (28; 30; 130, 230),
in which a protective layer is preferably arranged on the grid of the sensor, in order to arrange the one or the plurality of phase objects thereon, or
which preferably further comprises a container for accommodating the one or the plurality of phase objects, wherein the container is or can be arranged in the light path between the light source (37) and the grid and/or in the light path of the illuminating light scattered at the grid, wherein the sensor with the grid accommodated in or on the receptacle is located outside of the container.

## Revendications

1. Procédé d'examen d'un ou plusieurs objets de phase (4 ; 33 ; 233), une grille constituée d'éléments (10 ; 10' ; 10a-d) étant utilisée, qui est éclairée avec une lumière (20 ; 20a-d) d'une source de lumière (37), dont la longueur de cohérence est supérieure à la distance moyenne entre des éléments (10 ; 10' ; 10a-d) adjacents de la grille, une image de diffraction de la lumière d'éclairage diffusée au niveau de la grille étant générée, le ou les objets de phase (4 ; 33 ; 233) étant placés dans le trajet lumineux entre la source lumière (37) et la grille et/ou dans le trajet lumineux de la lumière d'éclairage diffusée au niveau de la grille, au moins une partie de l'image de diffraction étant détectée directement ou après interaction avec d'autres composants optiques par un détecteur optique (38), le détecteur optique (38) convertissant l'image de diffraction détectée ou la partie détectée de l'image de diffraction en un signal, ce signal étant analysé afin d'en déduire des informations concernant le ou les objets de phase (4 ; 33 ; 233).

2. Procédé selon la revendication 1, dans lequel le détecteur optique (38) détecte une série temporelle d'images de diffraction ou des parties d'images de diffraction, et la convertit en une séquence de signaux, la séquence de signaux étant analysée afin d'en déduire des informations concernant le ou les objets de phase (4 ; 33 ; 233), et/ou dans lequel au moins deux parties de l'image de diffraction sont détectées simultanément par au moins deux détecteurs optiques physiquement séparés entre eux et converties en signaux, ces signaux étant analysés afin d'en déduire des informations concernant le ou les objets de phase.

3. Procédé selon la revendication 1 ou 2, dans lequel au moins l'intensité d'un maximum principal de l'image de diffraction est détectée par le détecteur optique (38) et/ou dans lequel, entre la grille et le ou les objets de phase, une couche protectrice transparente est disposée et/ou dans lequel la grille et les objets de phase sont distants entre eux, les objets de phase étant de préférence logés dans un récipient séparé de la grille.

4. Procédé selon l'une des revendications précédentes, dans lequel les objets de phase (4) sont des cellules biologiques ou des petits animaux et/ou dans lequel, sur la base du signal, une modification du ou des objets de phase (4 ; 33 ; 233) est déterminée, et/ou dans lequel le ou les objets de phase (4) sont exposés en outre à une substance et/ou à un stimulus physique et/ou dans lequel, sur la base du signal, une variation de la superficie et/ou du volume du ou des objets de phase (233), un processus au sein du ou des objets de phase, une variation de la densité du ou des objets de phase par rapport à la grille est déterminée, et/ou, sur la base du signal, un degré de mélange d'au moins deux objets de phase (33) est déterminée et/ou dans lequel, sur la base du signal, un degré de recouvrement de la grille par le ou les objets de phase (4), un nombre d'objets de phase (4) et/ou une variation du de degré de recouvrement ou du nombre d'objets de phase (4) est déterminée.

5. Procédé selon l'une des revendications précédentes, dans lequel l'image de diffraction ou la partie de l'image de diffraction est détectée au moins partiellement en transmission à travers la grille ou dans lequel l'image de diffraction ou la partie de l'image de diffraction est détectée au moins partiellement en réflexion au niveau de la grille.

6. Procédé selon l'une des revendications précédentes, dans lequel les éléments de la grille sont disposés de manière périodique ou quasi-périodique et/ou dans lequel la grille est une grille bidimensionnelle.

7. Procédé selon la revendication 2 ou une revendication dépendante de celle-ci, dans lequel la séquence temporelle comprend au moins trois, plus particulièrement au moins 20 et de préférence au moins 100 images de diffraction ou parties détectées d'images de diffraction détectées successivement et/ou dans lequel, à l'aide d'un traitement de la séquence de signaux, une corrélation, plus particulièrement, une analyse de Fourier de la séquence temporelle des images de diffraction est effectuée et/ou dans lequel l'évolution en fonction du temps d'une intensité d'une portion des images de diffraction et/ou la fluctuation en fonction du temps de l'intensité d'une portion des images de diffraction est déterminée.

8. Procédé selon l'une des revendications précédentes, dans lequel, à l'aie du signal, une caractéristique du ou des objets de phase (4 ; 33 ; 233) est déterminée en et la caractéristique déterminée est comparée avec des entrées dans une base de données afin d'en déduire les informations concernant le ou les objets de phase, plus particulièrement un degré de recouvrement de la grille par le ou les objets de phase, un nombre d'objets de phase (4) et/ou une variation du degré de recouvrement ou du nombre d'objets de phase (4) et/ou un mouvement, une mobilité, un type, une forme, une modification de la superficie, une modification de la forme, un indice de réfraction, une variation de la densité, une modification du volume, une force exercée sur le ou les objets de phase, une force exercée par le ou les objets de phase, une substance à laquelle le ou les objets de phase sont exposés, un stimulus physique auquel le ou les objets de phase sont exposés, un degré de mélange et/ou une composition chimique du ou des objets de phase et/ou dans lequel, sur ou sous la grille, est disposée une matrice tridimensionnelle sur, sous ou dans laquelle sont disposés le ou les objets de phase.

9. Dispositif d'examen d'un ou de plusieurs objets de phase (4;33 ; 233) comprenant :

un logement (28 ; 30 ; 130, 230) pour le logement d'un capteur (24) avec une grille constituée d'éléments (10 ; 10' ; 10a-d),
une source de lumière (37), qui est ou peut être disposée de façon à ce qu'elle puisse éclairer un capteur (24) disposé dans ou sur le logement (28 ; 30 ; 130, 230) de façon à ce que, grâce à la lumière (25 ; 25a-d), diffusée au niveau de la grille, de la source de lumière (37), une image de diffraction soit générée et un ou plusieurs objets de phase (4 ; 33 ; 233) sont disposés dans le trajet lumineux entre la source de lumière (37) et la grille et/ou dans le trajet lumineux de la lumière d'éclairage diffusée au niveau de la grille,
un détecteur optique (38) qui est conçu pour détecter une partie de l'image de diffraction directement ou après interaction avec d'autre composants optiques et la convertir en un signal et
un circuit d'analyse (36), qui est couplé au détecteur optique (38) et qui reçoit le signal, le circuit d'analyse (36) étant conçu pour déterminer, à partir du signal, des informations concernant du ou des objets de phase (4 ; 33 ; 233).

10. Dispositif selon la revendication 9, dans lequel le détecteur optique (38) comprend un capteur d'image et/ou un photodétecteur et/ou dans lequel le détecteur optique (38) est disposé pour détecter au moins l'intensité d'un maximum principal de l'image de diffraction.

11. Dispositif selon la revendication 9 ou 10, dans lequel le détecteur optique (38) est conçu pour détecter une séquence temporelle d'images de diffraction ou de parties d'images de diffraction et pour la convertir en une séquence de signaux, le circuit d'analyse (36) étant conçu pour déterminer, à partir de la séquence de signaux, les informations concernant le ou les objets de phase (4 ; 33 ; 233), la séquence temporelle comprenant au moins trois, plus particulièrement au moins 20 et de préférence au moins 100 images de diffraction ou parties détectées d'images de diffraction détectées successivement et/ou le circuit d'analyse (36) étant de préférence conçu pour effectuer, grâce à un traitement de la séquence des signaux, une corrélation, plus particulièrement une analyse de Fourier de la séquence des images de diffraction détectées ou des parties détectées des images de diffraction.

**12.** Dispositif selon l'une des revendications 9 à 11, le détecteur optique étant un premier détecteur optique, qui est conçu pour détecter une première partie de l'image de diffraction et la convertir en un premier signal et le dispositif comprenant un deuxième détecteur optique, qui est séparé physiquement du premier détecteur optique et qui est conçu pour détecter une deuxième partie de l'image de diffraction et la convertir en un deuxième signal, le circuit d'analyse étant couplé en outre au deuxième détecteur optique et recevant le deuxième signal et étant conçu pour déterminer, à partir du premier et du deuxième signal, les informations concernant le ou les objets de phase et/ou dans lequel le détecteur optique (38) est disposé pour détecter l'image de diffraction ou la partie de l'image de diffraction au moins partiellement en transmission à travers la grille et/ou au moins partiellement en réflexion au niveau de la grille.

**13.** Dispositif selon l'une des revendications 9 à 12, dans lequel le circuit d'analyse (36) est conçu pour déterminer, sur la base du signal, une modification du ou des objets de phase (4 ; 33 ; 233),
le circuit d'analyse (36) étant de préférence conçu pour déterminer, sur la base du signal, une modification de la superficie et/ou du volume du ou des objets de phase (233), un processus au sein du ou des objets de phase, une modification de la densité du ou des objets de phase et/ou un mouvement du ou des objets de phase par rapport à la grille et/ou dans lequel le circuit d'analyse (36) est de préférence conçu pour déterminer, sur la base du signal, un degré de mélange d'au moins deux objets de phase (33) et/ou dans lequel le circuit d'analyse (36) est conçu pour déterminer, sur la base du signal, un degré d'un recouvrement de la grille par le ou les objets de phase (4), un nombre d'objets de phase (4) et/ou une variation du degré de recouvrement ou du nombre d'objets de phase (4).

**14.** Dispositif selon l'une des revendications 9 à 13, qui comprend en outre une mémoire de données avec une base de données enregistrée dans celle-ci ou qui peut être couplée avec celle-ci, et dans lequel le circuit d'analyse (36) est conçu pour déterminer, à l'aide du signal, une caractéristique du ou des objets de phase (4 ; 33 ; 233) et pour comparer la caractéristique déterminée avec des entrées dans la base de données, afin de déterminer les informations concernant le ou les objets de phase, plus particulièrement un degré de recouvrement de la grille par le ou les objets de phase, un nombre d'objets de phase (4), une variation du degré de recouvrement ou du nombre d'objets de phase (4) et/ou un mouvement, une mobilité, un type, une forme, une modification de la superficie, une modification de la forme, un indice de réfraction, une variation de densité, une modification du volume, une force exercée sur le ou les objets de phase, une force exercée par le ou les objets de phase, une substance à laquelle le ou les objets de phase sont exposés, un stimulus physique auquel le ou les objets de phase sont exposés, un degré de mélange et/ou une composition chimique du ou des objets de phase et/ou dans lequel le composant optique mentionné comprend une optique de Fourier qui est disposée afin de convertir l'image de diffraction ou la partie de l'image de diffraction en une image réelle d'une disposition du ou des objets de phase (4 ; 33 ; 233).

**15.** Dispositif selon l'une des revendications 9 à 14, qui contient en outre un capteur logé dans ou sur le logement (28 ; 30 ; 130, 230) avec une grille constituée d'éléments (10 ; 10' ; 10a-d), dans lequel, de préférence, une couche protectrice est disposée sur la grille du capteur, afin de disposer dessus le ou les objets de phase, ou qui comprend de préférence en outre un récipient pour le logement du ou des objets de phase, le récipient étant ou pouvant être disposé dans le trajet lumineux entre la source de lumière (37) et la grille et/ou dans le trajet lumineux de la lumière d'éclairage diffusée au niveau de la grille, le capteur logé dans ou sur le logement avec la grille se trouvant à l'extérieur du récipient.

Profilansicht     Substrat    10

1

Draufsicht     10

1

Fig. 1

Substrat    10'

10'

Fig. 2

EP 2 871 463 B1

Fig. 3

Fig. 4

EP 2 871 463 B1

Fig. 5

EP 2 871 463 B1

a)

b)

Fig. 6

Fig. 7

EP 2 871 463 B1

Fig. 8

Fig. 10a

Fig. 10b

Fig. 9a

Fig. 9b

Hellfeld Transmissionsmikroskopie

Monochromatische Reflexionmikroskopie

Fig. 11b

Fig. 11a

Fig. 12

Fig. 13

EP 2 871 463 B1

Fig. 14

Fig. 15b

Fig. 15a

Fig. 15c

Fig. 16

Fig. 17

Fig. 18

Fig. 19

a)                                                    b)

# Fig. 19

c)

# Fig. 19

## d)

**EP 2 871 463 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011050493 A1
- US 20050068543 A1
- DE 102010007365 A1
- US 2012231489 A1
- US 2002037593 A1